# EUROPÄISCHE PATENTSCHRIFT

(11) **EP 2 478 097 B1**
(45) Veröffentlichungstag und Bekanntmachung des Hinweises auf die Patenterteilung: **15.11.2017**
(21) Anmeldenummer: 10760643.6
(22) Anmeldetag: 15.09.2010
(51) Int. Cl.: C12N 9/54, C11D 3/36, C11D 3/386

(54) **LAGERSTABILES FLÜSSIGES WASCH- ODER REINIGUNGSMITTEL MIT PROTEASEN**
STABLE LIQUID WASHING OR CLEANING AGENT CONTAINING A PROTEASE
LESSIVE OU DÉTERGENT LIQUIDE STABLE AU STOCKAGE CONTENANT DES PROTÉASES

(30) Priorität: 16.09.2009 DE 102009029513
(43) Veröffentlichungstag der Anmeldung: 25.07.2012
(73) Patentinhaber: BASF SE, 67056 Ludwigshafen am Rhein (DE)
(72) Erfinder: WIELAND, Susanne, 41541 Zons/Dormagen (DE); SIEGERT, Petra, 42781 Haan (DE); SPITZ, Astrid, 47441 Moers (DE); MAURER, Karl-Heinz, 40699 Erkrath (DE); O'CONNELL, Timothy, 40545 Düsseldorf (DE); PRÜSER, Inken, 40215 Düsseldorf (DE); SCHIEDEL, Marc-Steffen, 40597 Düsseldorf (DE); EITING, Thomas, 40589 Düsseldorf (DE); SENDOR-MÜLLER, Dorota, 40589 Düsseldorf (DE); BASTIGKEIT, Thorsten, 42279 Wuppertal (DE); BENDA, Konstantin, 40217 Düsseldorf (DE); MÜLLER, Sven, 47051 Duisburg (DE)
(74) Vertreter: BASF IP Association
(86) Internationale Anmeldenummer: PCT/EP2010/063556
(87) Internationale Veröffentlichungsnummer: WO 2011/032988

(56) Entgegenhaltungen:
- WO-A1-00/36069
- WO-A1-95/23221
- WO-A2-02/088340
- WO-A2-2005/118793

### SEQUENCE LISTING

<110> Henkel AG & Co. KGaA
<120> Lagerstabiles füssiges Wasch- oder Reinigungsmittel enthaltend Proteasen
<130> H 08707 PCT
<150> DE 102009029513
   <151> 2009-09-16
<160> 8
<170> PatentIn version 3.3
<210> 1
   <211> 269
   <212> PRT
   <213> Bacillus lentus
<400> 1
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Arg Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 2
   <211> 269
   <212> PRT
   <213> Bacillus lentus
<400> 2
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Glu Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 3
   <211> 269
   <212> PRT
   <213> Bacillus lentus
<400> 3
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Asp Gly Ala Ile Ser Ser Ile Ala Gln Gly Leu Glu Trp Ala
   100 105 110
   Gly Asn Asn Gly Met His Val Ala Asn Leu Ser Leu Gly Ser Pro Ser
   115 120 125
   Pro Ser Ala Thr Leu Glu Gln Ala Val Asn Ser Ala Thr Ser Arg Gly
   130 135 140
   Val Leu Val Val Ala Ala Ser Gly Asn Ser Gly Ala Ser Ser Ile Ser
   145 150 155 160
   Tyr Pro Ala Arg Tyr Ala Asn Ala Met Ala Val Gly Ala Thr Asp Gln
   165 170 175
   Asn Asn Asn Arg Ala Ser Phe Ser Gln Tyr Gly Ala Gly Leu Asp Ile
   180 185 190
   Val Ala Pro Gly Val Asn Val Gln Ser Thr Tyr Pro Gly Ser Thr Tyr
   195 200 205
   Ala Ser Leu Asn Gly Thr Ser Met Ala Thr Pro His Val Ala Gly Ala
   210 215 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 4
   <211> 269
   <212> PRT
   <213> Bacillus lentus
<400> 4
   Ala Gln Ser Val Pro Trp Gly Ile Ser Arg Val Gln Ala Pro Ala Ala
   1 5 10 15
   His Asn Arg Gly Leu Thr Gly Ser Gly Val Lys Val Ala Val Leu Asp
   20 25 30
   Thr Gly Ile Ser Thr His Pro Asp Leu Asn Ile Arg Gly Gly Ala Ser
   35 40 45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Asn Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 5
   <211> 269
   <212> PRT
   <213> Bacillus lentus
<400> 5
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Gln Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 6
   <211> 269
   <212> PRT
   <213> Bacillus lentus
<400> 6
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Ala Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225 230 235 240
   Arg Asn His Leu Lys Asn Thr Ala Thr Ser Leu Gly Ser Thr Asn Leu
   245 250 255
   Tyr Gly Ser Gly Leu Val Asn Ala Glu Ala Ala Thr Arg
   260 265
<210> 7
   <211> 269
   <212> PRT
   <213> Bacillus lentus
<400> 7
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Gly Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 8
   <211> 269
   <212> PRT
   <213> Bacillus lentus
<400> 8
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Ser Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265

## Beschreibung

Die Erfindung liegt auf dem Gebiet der flüssigen Wasch- und Reinigungsmittel. Die Erfindung betrifft insbesondere die Verwendung von definierten Proteasen zur Bereitstellung einer proteolytischen Aktivität in einem flüssigen Wasch- oder Reinigungsmittel, in Kombination mit einem Phosphonat, und schlägt ferner Verfahren vor, in denen solche Mittel angewendet werden.

Für Wasch- und Reinigungsmittel werden bislang bevorzugt Proteasen vom Subtilisin-Typ eingesetzt. Die in den aus dem Stand der Technik bekannten Wasch- oder Reinigungsmitteln eingesetzten Proteasen stammen entweder ursprünglich aus Mikroorganismen, beispielsweise der Gattungen Bacillus, Streptomyces, Humicola, oder Pseudomonas, und/oder werden nach an sich bekannten biotechnologischen Verfahren durch geeignete Mikroorganismen produziert, beispielsweise durch transgene Expressionswirte der Gattungen Bacillus oder durch filamentöse Pilze.

Insbesondere in modernen Flüssigwaschmitteln, aber auch in Reinigungsmitteln, sind Phosphonate enthalten. Sie werden beispielsweise als Komplexbildner, zur Verhinderung von Ausfällungen oder als Bleichmittelstabilisator eingesetzt. Als Komplexbildner dienen sie beispielsweise als Wasserenthärter. Sie können Kationen wie Ca² in der Losung ummanteln und damit das chemische Verhalten des Kations verändern. Im Fall von Calcium verschwindet die Eigenschaft Wasserhärte zu bilden. Auch andere Kationen können komplexiert und damit vor chemischen Reaktionen geschützt werden. Sie können ferner als Korrosionsinhibitoren mitwirken oder als Stabilisator für Peroxide, insbesondere in Bleichmitteln, dienen.

In der internationalen Patentanmeldung WO 95/23221 sind Proteasen bzw. Protease-Varianten vom Subtilisin-Typ aus Bacillus lentus DSM 5483 offenbart, die für den Einsatz in Wasch- oder Reinigungsmitteln geeignet sind. Unter diesen Proteasen ist auch eine, die einen Aminosäureaustausch R99E, A, S oder G aufweisen kann. Ferner ist offenbart, dass die Waschmittel ein Phosphonat, insbesondere ein Polyphosphonat, enthalten können. Die Waschmittel können fest oder flüssig sein. Jedoch geht aus dieser Schrift kein konkretes flüssiges Wasch- bzw. Reinigungsmittel hervor, das zwingend ein Phosphonat in Kombination mit einer derartigen Protease enthält.

Ein Nachteil von proteasehaltigen flüssigen Wasch- und Reinigungsmitteln aus dem Stand der Technik ist, dass sie oftmals keine zufrieden stellende proteolytische Aktivität aufweisen, insbesondere nicht bei niedrigen Temperaturen, beispielsweise zwischen 10°C und 50°C, insbesondere zwischen 10°C und 40°C oder zwischen 20°C und 40°C, und das Wasch- oder Reinigungsmittel daher keine optimale Reinigungsleistung zeigt. Ein weiterer Nachteil von proteasehaltigen flüssigen Wasch- und Reinigungsmitteln aus dem Stand der Technik ist, dass sie nicht ausreichend lagerstabil sind und sie demnach bereits nach kurzer Zeit ein erhebliches Maß an proteolytischer Aktivität einbüßen.

Der vorliegenden Erfindung liegt die Aufgabe zugrunde, die genannten Nachteile zu überwinden und flüssige Wasch- oder Reinigungsmittel bereitzustellen, die eine vorteilhafte proteolytische Aktivität aufweisen, insbesondere bei Temperaturen wie vorstehend genannt, und zudem verbessert lagerstabil sind.

Ein Gegenstand der Erfindung ist daher die Verwendung einer Protease,
(a1) die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Glutaminsäure (E) oder Asparaginsäure (D) aufweist, zur Bereitstellung einer proteolytischen Aktivität in einem flüssigen Wasch- oder Reinigungmittel, welches ferner ein Phosphonat in einer Menge von 0,01 bis 4 Gew.% umfasst.

Überraschenderweise wurde festgestellt, dass ein flüssiges Wasch- oder Reinigungsmittel, welches die Kombination einer solchen Protease mit einem Phosphonat enthält, vorteilhafte Reinigungsleistungen an protease-sensitiven Anschmutzungen aufweist und zudem vorteilhaft lagerstabil ist. In einer bevorzugte Ausführungsformen der Erfindung sind die Mittel dadurch gekennzeichnet, dass sie eine vorteilhafte Reinigungsleistung hinsichtlich mindestens einer protease-sensitiven Anschmutzung bei Temperaturen zwischen 10°C und 60°C, vorzugsweise auch bei niedrigen Temperaturen, beispielsweise zwischen 10°C und 50°C, zwischen 10°C und 40°C oder zwischen 20°C und 40°C zeigen. In einer Ausführungsform ermöglicht das Mittel daher eine verbesserte Entfernung von mindestens einer, bevorzugt von mehreren protease-sensitiven Anschmutzungen auf Textilien und/oder harten Oberflächen, beispielsweise Geschirr. Hinsichtlich der einleitend erwähnten internationalen Patentanmeldung WO 95/23221 handelt es bei der vorliegenden Erfindung daher um eine besonders vorteilhafte Auswahl, die zum Erhalt eines leistungsfähigen und lagerstabilen flüssigen Waschmittels bzw. flüssigen Reinigungsmittels führt.

Unter Reinigungsleistung wird im Rahmen der Erfindung die Aufhellungsleistung an einer oder mehreren Anschmutzungen, insbesondere Wäscheanschmutzungen oder Geschirranschmutzungen, verstanden, die sensitiv sind für den Abbau durch die Protease. Beispiele für solche Wäscheanschmutzungen sind Blut-Milch/Tusche auf Baumwolle, Voll-Ei/Pigment auf Baumwolle, Schokolade-Milch/Tusche auf Baumwolle, Erdnuss Öl-Pigment/Tusche auf Polyester/Baumwolle, Gras auf Baumwolle oder Kakao auf Baumwolle, insbesondere derart wie weiter unten angegeben. Beispiele für solche Geschirranschmutzungen sind Milch, Hackfleisch oder Eigelb. Im Rahmen der Erfindung weist sowohl das Wasch- oder Reinigungsmittel, welches die Protease umfasst bzw. die durch dieses Mittel gebildete Wasch- bzw. Reinigungsflotte, als auch die Protease selbst eine jeweilige Reinigungsleistung auf. Die Reinigungsleistung des hydrolytischen Enzyms trägt somit zur Reinigungsleistung des Mittels bzw. der durch das Mittel gebildeten Wasch- bzw. Reinigungsflotte bei. Die Reinigungsleistung wird bevorzugt ermittelt wie weiter unten angegeben.

Unter Wasch- bzw. Reinigungsflotte wird diejenige das Wasch- oder Reinigungsmittel enthaltende Gebrauchslösung verstanden, die auf Textilien oder Gewebe (Waschflotte) bzw. harte Oberflächen (Reinigungsflotte) einwirkt und damit mit den auf Textilien bzw. Geweben oder harten Oberflächen vorhandenen Anschmutzungen in Kontakt kommt. Üblicherweise entsteht die Wasch- bzw. Reinigungsflotte, wenn der Wasch- oder Reinigungsvorgang beginnt und das Wasch- oder Reinigungsmittel beispielsweise in einer Waschmaschine, Geschirrspülmaschine oder in einem anderen geeigneten Behältnis mit Wasser verdünnt wird.

In einer Ausführungsform der Erfindung enthält das Wasch- oder Reinigungsmittel Protease umfassend eine Aminosäuresequenz, die zu der in SEQ ID NO. 1 angegebenen Aminosäuresequenz zu mindestens 80% identisch ist und die an Position 99 in der Zählung gemäß SEQ ID NO. 1 die Aminosäure Glutaminsäure (E) oder Asparaginsäure (D) aufweist. Zunehmend bevorzugt ist die Aminosäuresequenz zu mindestens 81%, 82%, 83%, 84%, 85%, 86%, 87%, 88%, 89%, 90%, 91 %, 92%, 93%, 94%, 95%, 96%, 97%, 98% und ganz besonders bevorzugt zu 99% identisch zu der in SEQ ID NO. 1 angegebenen Aminosäuresequenz. SEQ ID NO. 1 ist die Sequenz der reifen (maturen) alkalischen Protease aus Bacillus lentus DSM 5483, die offenbart ist in der internationalen Patentanmeldung WO 92/21760, und auf deren Offenbarung hiermit ausdrücklich Bezug genommen wird.

Die in einem offenbarungsgemäßen Wasch- oder Reinigungsmittel enthaltene Protease umfasst eine Aminosäuresequenz, die zu der in SEQ ID NO. 1 angegebenen Aminosäuresequenz zu mindestens 80% identisch ist und die an Position 99 in der Zählung gemäß SEQ ID NO. 1 die Aminosäure Asparagin (N) oder Glutamin (Q) oder die Aminosäure Alanin (A) oder Glycin (G) oder Serin (S) aufweist. Zunehmend bevorzugt ist die Aminosäuresequenz zu mindestens 81%, 82%, 83%, 84%, 85%, 86%, 87%, 88%, 89%, 90%, 91%, 92%, 93%, 94%, 95%, 96%, 97%, 98% und ganz besonders bevorzugt zu 99% identisch zu der in SEQ ID NO. 1 angegebenen Aminosäuresequenz. SEQ ID NO. 1 ist die Sequenz der reifen (maturen) alkalischen Protease aus Bacillus lentus DSM 5483, die offenbart ist in der internationalen Patentanmeldung WO 92/21760, und auf deren Offenbarung hiermit ausdrücklich Bezug genommen wird.

Erfindungsgemäß hat sich gezeigt, dass durch den Zusatz einer solchen Protease zu einem flüssigen Wasch- oder Reinigungsmittel, welches ein Phosphonat enthält, ein besonders lagerstabiles Flüssigwaschmittel erhalten wird, insbesondere hinsichtlich dessen verbleibender proteolytischer Aktivität nach Lagerung, insbesondere nach einer Lagerdauer von 1 bis 5 Wochen, 1 bis 4 Wochen, 1,5 bis 3 Wochen und besonders bevorzugt nach 2 Wochen.

Eine in einem Wasch- oder Reinigungsmittel enthaltene Protease weist eine proteolytische Aktivität auf, das heißt, sie ist zur Hydrolyse von Peptidbindungen eines Polypeptids bzw. Proteins befähigt. Sie ist daher ein Enzym, welches die Hydrolyse von Peptidbindungen katalysiert und dadurch in der Lage ist, Peptide oder Proteine zu spalten, insbesondere ein Subtilisin.

In einer weiteren Ausführungsform der Erfindung ist das Wasch- oder Reinigungsmittel dadurch gekennzeichnet, dass die Protease ferner in der Zählung gemäß SEQ ID NO. 1 mindestens eine der folgenden Aminosäuren aufweist:
(a) Threonin an Position 3 (3T),
(b) Isoleucin an Position 4 (4I),
(c) Alanin, Threonin oder Arginin an Position 61 (61A, 61T oder 61R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (199I),
(h) Asparaginsäure, Glutaminsäure oder Glycin an Position 211 (211D, 211E oder 211G),
(i) Kombinationen der Aminosäuren (a) bis (h).

Neben einer der genannten Aminosäuren an Position 99 weist die Protease daher eine oder mehrere der vorstehend genannten Aminosäuren an den jeweiligen Positionen auf. Diese Aminosäuren können weitere vorteilhafte Eigenschaften bewirken und/oder bereits vorhandene Eigenschaften noch verstärken. Insbesondere bewirken die vorstehend genannten Aminosäuren eine Steigerung der proteolytischen Aktivität und/oder der Stabilität der Protease in einem flüssigen Wasch- oder Reinigungsmittel bzw. in der durch dieses Wasch- oder Reinigungsmittel gebildeten Waschflotte. Durch den Zusatz einer solchen Protease zu einem flüssigen Wasch- oder Reinigungsmittel, welches ein Phosphonat enthält, wird somit ebenfalls ein besonders lagerstabiles Flüssigwaschmittel erhalten, insbesondere hinsichtlich dessen verbleibender proteolytischer Aktivität nach Lagerung, insbesondere nach einer Lagerdauer von 1 bis 5 Wochen, 1 bis 4 Wochen, 1,5 bis 3 Wochen und besonders bevorzugt nach 2 Wochen.

Die Aminosäurepositionen werden hierbei durch ein Alignment der Aminosäuresequenz der einzusetzenden Protease mit der Aminosäuresequenz der Protease aus Bacillus lentus, wie sie in SEQ ID NO. 1 angegeben ist, definiert. Da die Protease aus Bacillus lentus im Stand der Technik ein wichtiges Referenzmolekül zur Beschreibung von Proteasen und von Aminosäureveränderungen darstellt ist es vorteilhaft, in der Zuordnung der Aminosäurepositionen auf die Zählung der Protease aus Bacillus lentus (SEQ ID NO. 1) Bezug zu nehmen. Weiterhin richtet sich die Zählung nach dem reifen (maturen) Protein. Diese Zuordnung ist insbesondere auch anzuwenden, wenn die Aminosäuresequenz der einzusetzenden Protease eine höhere Zahl von Aminosäurenresten umfasst als die Protease aus Bacillus lentus gemäß SEQ ID NO. 1.

Ausgehend von den genannten Positionen in der Aminosäuresequenz der Protease aus Bacillus lentus sind die Aminosäurepositionen in einer erfindungsgemäß einzusetzenden Protease diejenigen, die eben diesen Positionen in einem Alignment zugeordnet sind.

Neben Position 99 besonders vorteilhafte Positionen sind demnach die Positionen 3, 4, 61, 154, 188, 193, 199 und 211, zuzuordnen in einem Alignment mit SEQ ID NO. 1 und damit in der Zählung gemäß SEQ ID NO. 1. In den genannten Positionen liegen in dem Wildtypmolekül der Protease aus Bacillus lentus folgende Aminosäurereste: S3, V4, G61, S154, A188, V193, V199, und L211. Besonders bevorzugt sind die Aminosäuren 3T, 4I, 61A, 154D, 154E, 211D, 211G und 211E, sofern die entsprechenden Positionen in einer erfindungsgemäß einzusetzenden Protease nicht schon natürlicherweise von einer dieser bevorzugten Aminosäuren eingenommen werden. Die Austausche 3T und 4I führen beispielsweise über einen Stabilisierungseffekt auf das Molekül zu einer Verbesserung der Lagerstabilität und der Reinigungsleistung der Protease und damit zu einer verbesserten Reinigungsleistung eines Phosphonat-haltigen flüssigen Wasch- oder Reinigungsmittels, das die Protease enthält.

Auf Grund der vorstehend genannten, für die jeweilige Position vorgesehenen Aminosäuren können sich weitere Sequenzabweichungen von SEQ ID NO. 1 ergeben, sofern SEQ ID NO. 1 eine andere Aminosäure in der jeweiligen Position aufweist. In Abhängigkeit von der Anzahl der vorliegenden Sequenzabweichungen von SEQ ID NO.1 ergeben sich daher unterschiedliche maximale Identitätswerte, die eine erfindungsgemäß einzusetzende Protease zu SEQ ID NO. 1 aufweisen kann, selbst wenn sie in allen übrigen Aminosäuren mit SEQ ID NO. 1 übereinstimmen sollte. Dieser Sachverhalt ist für jede mögliche Kombination der vorgeschlagenen Aminosäuren im Einzelfall zu berücksichtigen und ist ferner auch abhängig von der Länge der Aminosäuresequenz der Protease. Beispielsweise betragt die maximale Identität bei einer, zwei, drei, vier, fünf, sechs, sieben, acht oder neun Sequenzveränderungen 99,63%, 99,26%, 98,88%, 98,51%, 98,14%, 97,77%, 97,40%, 97,03% bzw. 96,65% bei einer 269 Aminosäuren langen Aminosäuresequenz, beziehungsweise 99,64%, 99,27%, 98,91%, 98,55%, 98,18%, 97,82%, 97,45%, 97,09% bzw. 96,73% bei einer 275 Aminosäuren langen Aminosäuresequenz.

Die Bestimmung der Identität von Nukleinsäure- oder Aminosäuresequenzen erfolgt durch einen Sequenzvergleich. Solch ein Vergleich erfolgt dadurch, dass ähnliche Abfolgen in den Nukleotidsequenzen oder Aminosäuresequenzen einander zugeordnet werden. Dieser Sequenzvergleich erfolgt vorzugsweise basierend auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mal. Biol. 215:403- 410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden üblicherweise mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mal. Biol. 302, 205-217) oder Programme, die auf diesen Programmen bzw. Algorithmen basieren. Häufig genutzt werden beispielsweise Clustal (vgl. beispielsweise Chenna et al. (2003): Multiple sequence alignment with the Clustal series of programs. Nucleic Acid Research 31, 3497-3500) oder T-Coffee (vgl. beispielsweise Notredame et al. (2000): T-Coffee: A novel method for multiple sequence alignments. J. Mol. Biol. 302, 205-217) sowie BLAST oder FASTA für die Datenbanksuche, beziehungsweise Programme, die auf diesen Programmen bzw. Algorithmen basieren. Im Rahmen der vorliegenden Erfindung werden Sequenzvergleiche und Alignments bevorzugt mit dem Computer-Programm Vector NTI® Suite 10.3 (Invitrogen Corporation, 1600 Faraday Avenue, Carlsbad, Kalifornien, USA) mit den vorgegebenen Default-Parametern erstellt.

Solch ein Vergleich erlaubt eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure-oder Aminosäuresequenzen sind daher durch Übereinstimmungen in den Sequenzen definiert. Solche Bereiche weisen oftmals identische Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäure- oder Aminosäuresequenz.

In einer weiteren Ausführungsform dieses Erfindungsgegenstandes ist das Wasch- oder Reinigungsmittel dadurch gekennzeichnet, dass die Protease eine Aminosäuresequenz umfasst, die zu der in SEQ ID NO. 1 angegebenen Aminosäuresequenz wie vorstehend angegeben identisch ist und die aus einer Protease gemäß SEQ ID NO. 1 durch ein- oder mehrfache konservative Aminosäuresubstitution erhalten wird bzw. erhältlich ist. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T.

In einer weiteren Ausführungsform der Erfindung ist ein Wasch- oder Reinigungsmittel ferner dadurch gekennzeichnet, dass seine Reinigungsleistung mindestens derjenigen eines Wasch- bzw. Reinigungsmittels entspricht, das eine Protease beinhaltet, die eine Aminosäuresequenz umfasst, die der in SEQ ID NO. 2 oder SEQ ID NO. 3 angegebenen Aminosäuresequenz entspricht, wobei die Reinigungsleistung in einem Waschsystem bestimmt wird, das ein Waschmittel in einer Dosierung zwischen 4,0 und 8,0 Gramm pro Liter Waschflotte sowie die Protease enthält, wobei die zu vergleichenden Proteasen aktivitätsgleich eingesetzt sind und die Reinigungsleistung gegenüber einer oder mehrerer der Anschmutzungen Blut-Milch/Tusche auf Baumwolle, Voll-Ei/Pigment (Ganzei/Ruß) auf Baumwolle, Erdnuss Öl-Pigment/Tusche auf Polyester/Baumwolle und Gras auf Baumwolle, insbesondere gegenüber einer oder mehrerer der Anschmutzungen
- Blut-Milch/Tusche auf Baumwolle: Produkt Nr. C-05 erhältlich von CFT (Center For Testmaterials) B.V. Vlaardingen, Niederlande
- Voll-Ei/Pigment (Ganzei/Ruß) auf Baumwolle: Produkt Nr. 10N erhältlich von der Firma wfk Testgewebe GmbH; Brüggen-Bracht, Deutschland, oder Produkt C-S-37 erhältlich von CFT (Center For Testmaterials) B.V. Vlaardingen, Niederlande
- Erdnuss Öl-Pigment/Tusche auf Polyester/Baumwolle: Produkt Nr. PC-10 erhältlich von CFT (Center For Testmaterials) B.V. Vlaardingen, Niederlande
- Gras auf Baumwolle: Produkt Nr. 164 erhältlich von der Firma Eidgenössische Material- und Prüfanstalt (EMPA) Testmaterialien AG, St. Gallen, Schweiz,
bestimmt wird durch Messung des Weißheitsgrades der gewaschenen Textilien, der Waschvorgang für mindestens 30 Minuten, optional 60 Minuten, bei einer Temperatur von 20°C erfolgt und das Wasser eine Wasserhärte zwischen 15,5 und 16,5° (deutsche Härte) aufweist.

In einer weiteren Ausführungsform der Offenbarung ist ein Wasch- oder Reinigungsmittel ferner dadurch gekennzeichnet, dass seine Reinigungsleistung mindestens derjenigen eines Wasch- bzw. Reinigungsmittels entspricht, das eine Protease beinhaltet, die eine Aminosäuresequenz umfasst, die der in SEQ ID NO. 4 oder SEQ ID NO. 5 oder SEQ ID NO. 6 oder SEQ ID NO. 7 oder SEQ ID NO. 8 angegebenen Aminosäuresequenz entspricht, wobei die Reinigungsleistung in einem Waschsystem bestimmt wird, das ein Waschmittel in einer Dosierung zwischen 4,0 und 8,0 Gramm pro Liter Waschflotte sowie die Protease enthält, wobei die zu vergleichenden Proteasen aktivitätsgleich eingesetzt sind und die Reinigungsleistung gegenüber einer oder mehrerer der Anschmutzungen Blut- Milch/Tusche auf Baumwolle, Voll-Ei/Pigment (Ganzei/Ruß) auf Baumwolle, Erdnuss Öl-Pigment/Tusche auf Polyester/Baumwolle und Gras auf Baumwolle, insbesondere gegenüber einer oder mehrerer der Anschmutzungen
- Blut-Milch/Tusche auf Baumwolle: Produkt Nr. C-05 erhältlich von CFT (Center For Testmaterials) B.V. Vlaardingen, Niederlande
- Voll-Ei/Pigment (Ganzei/Ruß) auf Baumwolle: Produkt Nr. 10N erhältlich von der Firma wfk Testgewebe GmbH; Brüggen-Bracht, Deutschland, oder Produkt C-S-37 erhältlich von CFT (Center For Testmaterials) B.V. Vlaardingen, Niederlande
- Erdnuss Öl-Pigment/Tusche auf Polyester/Baumwolle: Produkt Nr. PC-10 erhältlich von CFT (Center For Testmaterials) B.V. Vlaardingen, Niederlande
- Gras auf Baumwolle: Produkt Nr. 164 erhältlich von der Firma Eidgenössische Material- und Prüfanstalt (EMPA) Testmaterialien AG, St. Gallen, Schweiz,
bestimmt wird durch Messung des Weißheitsgrades der gewaschenen Textilien, der Waschvorgang für mindestens 30 Minuten, optional 60 Minuten, bei einer Temperatur von 20°C erfolgt und das Wasser eine Wasserhärte zwischen 15,5 und 16,5° (deutsche Härte) aufweist.

Das Waschmittel für das Waschsystem ist ein flüssiges Waschmittel, das vorzugsweise wie in Tabelle 1 angegeben zusammengesetzt ist (alle Angaben in Gewichts-Prozent):

**Tabelle 1:**

| Inhaltsstoff | Gew.-% |
|---|---|
| C₁₂-C₁₈ Fettalkohol mit 7 EO | 6,40 |
| Lin. C₁₀-C₁₃ Alkylbenzolsulfonat (Na-Salz) | 5,35 |
| C₁₂-C₁₈ Fettsäure (Na-Salz) | 2,00 |
| Citronensäure (Na-Salz) | 1,20 |
| Phosphonat (Dequest® 2066) | 0,50 |
| Borsäure (Na-Salz) | 1,00 |
| Polyacrylat-Verdicker | 0,15 |
| Glycerin | 3,00 |
| Ethanol | 1,00 |
| Silikon-Entschäumer | 0,01 |
| Parfüm | 0,70 |
| Farbstoff, Konservierungsmittel | + |
| Wasser | Ad 100 |

Die bevorzugte Dosierung dieses flüssigen Waschmittels beträgt zwischen 7,0 und 7,5 Gramm pro Liter Waschflotte, insbesondere 7,4 Gramm pro Liter Waschflotte. Diesbezüglich wird das Gewicht des flüssigen Waschmittels bestimmt, so dass die Angaben bezogen sind auf dessen Gewicht. Bevorzugt wird gewaschen in einem pH-Wertebereich zwischen pH 8 und pH 10,5, bevorzugt zwischen pH 8 und pH 9.

Der Weißheitsgrad, d.h. die Aufhellung der Anschmutzungen, als Maß für die Reinigungsleistung wird bevorzugt mit optischen Messverfahren bestimmt, bevorzugt photometrisch. Ein hierfür geeignetes Gerät ist beispielsweise das Spektrometer Minolta CM508d. Üblicherweise werden die für die Messung eingesetzten Geräte zuvor mit einem Weißstandard, bevorzugt einem mitgelieferten Weißstandard, kalibriert.

Durch den aktivitätsgleichen Einsatz der jeweiligen Protease wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also beispielsweise die Reinigungsleistung an bestimmten Anschmutzungen, verglichen werden. Generell gilt, dass eine niedrige spezifische Aktivität durch Zugabe einer größeren Proteinmenge ausgeglichen werden kann. Verfahren zur Bestimmung der Proteaseaktivitäten sind dem Fachmann auf dem Gebiet der Enzymtechnologie geläufig und werden von ihm routinemäßig angewendet. Beispielsweise sind solche Verfahren offenbart in Tenside, Band 7 (1970), S. 125-132. Die Proteaseaktivität wird vorzugsweise in PE (Protease-Einheiten) angegeben. Beispielsweise geeignete Proteaseaktivitäten betragen 2,25, 5 oder 10 PE (Protease-Einheiten) pro ml Waschflotte. Die Proteaseaktivität ist jedoch nicht gleich Null.

Zahlreiche Proteasen und insbesondere Subtilisine werden als sogenannte Präproteine, also zusammen mit einem Propeptid und einem Signalpeptid gebildet, wobei die Funktion des Signalpeptids üblicherweise darin besteht, die Ausschleusung der Protease aus der sie produzierenden Zelle in das Periplasma oder das die Zelle umgebende Medium zu gewährleisten, und das Propeptid üblicherweise für die korrekte Faltung der Protease nötig ist. Das Signalpeptid und das Propeptid sind in der Regel der N-terminale Teil des Präproteins. Das Signalpeptid wird unter natürlichen Bedingungen durch eine Signalpeptidase von der übrigen Protease abgespalten. Anschließend erfolgt die durch das Propeptid unterstützte korrekte endgültige Faltung der Protease. Die Protease ist dann in ihrer aktiven Form und spaltet das Propeptid selbst ab. Nach der Abspaltung des Propeptids übt die dann reife (mature) Protease, insbesondere Subtilisin, ihre katalytische Aktivität ohne die ursprünglich vorhandenen N-terminalen Aminosäuren aus. Für technische Anwendungen allgemein und insbesondere im Rahmen der Erfindung sind die reifen (maturen) Proteasen, d.h. die nach ihrer Herstellung prozessierten Enzyme, gegenüber den Präproteinen bevorzugt. Die Proteasen können ferner von den sie produzierenden Zellen nach der Herstellung der Polypeptidkette modifiziert werden, beispielsweise durch Anknüpfung von Zuckermolekülen, Formylierungen, Aminierungen, usw. Solche Modifikationen sind posttranslationale Modifikationen und können, müssen jedoch nicht einen Einfluss auf die Funktion der Protease ausüben.

Ferner kann auch die reife Protease an ihrem N-terminalen und/oder C-terminalen Ende verkürzt sein, so dass eine gegenüber SEQ ID NO. 1 bzw. SEQ ID NO. 2 oder SEQ ID NO. 3 oder SEQ ID NO. 4 oder SEQ ID NO. 5 oder SEQ ID NO. 6 oder SEQ ID NO. 7 oder SEQ ID NO. 8 verkürzte Protease, also ein Fragment, in dem erfindungsgemäßen Wasch- oder Reinigungsmittel enthalten ist. Alle Identitätsangaben beziehen sich in diesem Fall auf denjenigen Bereich, in dem das jeweilige Fragment in einem Alignment SEQ ID NO. 1 zugeordnet ist. Das jeweilige Fragment beinhaltet aber in jedem Fall diejenige Position, die der Position 99 in einem Alignment mit SEQ ID NO. 1 zugeordnet ist, und weist an dieser Position eine entsprechende Aminosäure auf. Vorteilhafterweise beinhaltet es auch eine oder mehrere der weiteren vorstehend beschriebenen Positionen und weist dort eine oder mehrere entsprechende Aminosäuren auf. Ferner ist ein solches Fragment proteolytisch aktiv. Ein diesbezüglich weiter bevorzugtes Fragment umfasst eine Aminosäuresequenz, die über eine Länge von mindestens 50 oder mindestens 60, 70, 80, 90, 100, 110, 120, 130, 140, 150, 160, 170, 180, 190, 200, 210, 220, 230, 240, 250, 260, 265, 266, 267 oder 268 zusammenhangenden Aminosäurepositionen mit SEQ ID NO. 1 oder SEQ ID NO. 2 oder SEQ ID NO. 3 oder SEQ ID NO. 4 oder SEQ ID NO. 5 oder SEQ ID NO. 6 oder SEQ ID NO. 7 oder SEQ ID NO. 8 übereinstimmt unter Berücksichtigung der vorstehend genannten Aminosäuren für die Position 99 und optional ferner für die Positionen 3 und/oder 4 und/oder 61 und/oder 154 und/oder 188 und/oder 193 und/oder 199 und/oder 211. Besonders bevorzugt entspricht die Reinigungsleistung eines flüssigen Wasch- oder Reinigungsmittels mit einem solchen Fragment mindestens derjenigen eines Wasch- bzw. Reinigungsmittels, das eine Protease beinhaltet, die eine Aminosäuresequenz umfasst, die der in SEQ ID NO. 2 oder SEQ ID NO. 3 oder SEQ ID NO. 4 oder SEQ ID NO. 5 oder SEQ ID NO. 6 oder SEQ ID NO. 7 oder SEQ ID NO. 8 angegebenen Aminosäuresequenz entspricht, bestimmt wie vorstehend angegeben.

Ferner kann die in einem Mittel enthaltene Protease an Trägerstoffe adsorbiert und/oder in Hüllsubstanzen eingebettet sein, um sie gegen vorzeitige Inaktivierung zu schützen. In der Waschflotte, also unter Anwendungsbedingungen, wird die Protease dann freigesetzt und kann ihre proteolytische Wirkung entfalten.

In einem weiterer Gegenstand der Erfindung ist ein flüssiges Wasch- oder Reinigungsmittel dadurch gekennzeichnet, dass es umfasst:
(a) eine Protease, die ausgewählt ist aus der Gruppe bestehend aus
   a. Protease umfassend eine Aminosäuresequenz gemäß SEQ ID NO. 2 oder SEQ ID NO. 3;
   b. Protease, die eine gegenüber SEQ ID NO. 2 oder SEQ ID NO. 3 in mindestens einer Position veränderte Aminosäuresequenz umfasst, wobei die Veränderung in der Zählung gemäß SEQ ID NO. 1 ausgewählt ist aus der Gruppe bestehend aus:
      i. Threonin an Position 3 (3T),
      ii. Isoleucin an Position 4 (4I),
      iii. Alanin, Threonin oder Arginin an Position 61 (61A, 61T oder 61R),
      iv. Asparaginsäure oder Glutaminsäure an Position 154 (154D oder 154E),
      v. Prolin an Position 188 (188P),
      vi. Methionin an Position 193 (193M),
      vii. Isoleucin an Position 199 (199I),
      viii. Asparaginsäure, Glutaminsäure oder Glycin an Position 211 (211D, 211E oder 211G),
      ix. Kombinationen der Aminosäuren (i) bis (viii);
(b) ein Phosphonat.

Diese Proteasen werden ganz besonders bevorzugt in einem flüssigen Wasch- oder Reinigungsmittel eingesetzt. Sie werden ausgehend von SEQ ID NO. 1 erhalten durch die Substitution der Aminosäure Arginin an Position 99 durch die Aminosäure Glutaminsäure (E) oder Asparaginsäure (D) in der Zählung gemäß SEQ ID NO. 1. Diese Aminosäuresequenzen sind im Sequenzprotokoll als SEQ ID NO. 2 und SEQ ID NO. 3 angegeben. Ferner können diese Proteasen zusätzlich zu der für Position 99 vorgesehenen Aminosäure eine oder mehrere der vorstehend erläuterten Aminosäuren in den Positionen 3, 4, 61, 154, 188, 193, 199 und 211 aufweisen, zuzuordnen in einem Alignment mit SEQ ID NO. 1 und damit in der Zählung gemäß SEQ ID NO. 1. Die genannten Aminosäuren für diese Positionen bewirken auch bei diesen Proteasen weitere vorteilhafte Eigenschaften und/oder verstärken bereits vorhandene Eigenschaften noch. Insbesondere bewirken sie eine Steigerung der proteolytischen Aktivität und/oder der Stabilität der Protease in einem flüssigen Wasch- oder Reinigungsmittel bzw. in der durch dieses Wasch- oder Reinigungsmittel gebildeten Waschflotte. Alle vorstehenden Ausführungen - sofern anwendbar - gelten für diese besonders bevorzugten Proteasen entsprechend.

Offenbart ist weiterhin ein flüssiges Wasch- oder Reinigungsmittel umfassend:
(a) eine Protease, die ausgewählt ist aus der Gruppe bestehend aus
   a. Protease umfassend eine Aminosäuresequenz gemäß SEQ ID NO. 4 oder SEQ ID NO. 5 oder SEQ ID NO. 6 oder SEQ ID NO. 7 oder SEQ ID NO. 8;
   b. Protease, die eine gegenüber SEQ ID NO. 4 oder SEQ ID NO. 5 oder SEQ ID NO. 6 oder SEQ ID NO. 7 oder SEQ ID NO. 8 in mindestens einer Position veränderte Aminosäuresequenz umfasst, wobei die Veränderung in der Zählung gemäß SEQ ID NO. 1 ausgewählt ist aus der Gruppe bestehend aus:
      i. Threonin an Position 3 (3T),
      ii. Isoleucin an Position 4 (4I),
      iii. Alanin, Threonin oder Arginin an Position 61 (61A, 61T oder 61R),
      iv. Asparaginsäure oder Glutaminsäure an Position 154 (154D oder 154E),
      v. Prolin an Position 188 (188P),
      vi. Methionin an Position 193 (193M),
      vii. Isoleucin an Position 199 (199I),
      viii. Asparaginsäure, Glutaminsäure oder Glycin an Position 211 (211D, 211E oder 211G),
      ix. Kombinationen der Aminosäuren (i) bis (viii);
(b) ein Phosphonat.

Diese Proteasen werden ganz besonders bevorzugt in einem flüssigen Wasch- oder Reinigungsmittel eingesetzt. Sie werden ausgehend von SEQ ID NO. 1 erhalten durch die Substitution der Aminosäure Arginin an Position 99 durch die Aminosäure Asparagin (N) oder Glutamin (Q) oder die Aminosäure Alanin (A) oder Glycin (G) oder Serin (S) in der Zählung gemäß SEQ ID NO. 1. Diese Aminosäuresequenzen sind im Sequenzprotokoll als SEQ ID NO. 4, SEQ ID NO. 5, SEQ ID NO. 6, SEQ ID NO. 7 und SEQ ID NO. 8 angegeben. Ferner können diese Proteasen zusätzlich zu der für Position 99 vorgesehenen Aminosäure eine oder mehrere der vorstehend erläuterten Aminosäuren in den Positionen 3, 4, 61, 154, 188, 193, 199 und 211 aufweisen, zuzuordnen in einem Alignment mit SEQ ID NO. 1 und damit in der Zählung gemäß SEQ ID NO. 1. Die genannten Aminosäuren für diese Positionen bewirken auch bei diesen Proteasen weitere vorteilhafte Eigenschaften und/oder verstärken bereits vorhandene Eigenschaften noch. Insbesondere bewirken sie eine Steigerung der proteolytischen Aktivität und/oder der Stabilität der Protease in einem flüssigen Wasch- oder Reinigungsmittel bzw. in der durch dieses Wasch- oder Reinigungsmittel gebildeten Waschflotte. Alle vorstehenden Ausführungen - sofern anwendbar - gelten für diese besonders bevorzugten Proteasen entsprechend.

Phosphonate sind Salze und organische Verbindungen, insbesondere Ester, der Phosphonsäure. Als Salze existieren primäre (M'H₂PO₃ bzw. HP(O)(OH)(OM')) und sekundäre (M'₂HPO₃ bzw. HP(O)(OM')₂) Phosphonate, wobei M' für ein einwertiges Metall steht. Diese anorganischen Phosphonate und werden auch als primäre bzw. sekundäre Phosphite bezeichnet. Anorganische Phosphonate entstehen beispielsweise durch Umsetzung von Phosphonsäure HP(O)(OH)₂, insbesondere der stabilen tautomeren Form der Phosphorigsäure mit einem (primäre) oder zwei (sekundäre) Äquivalenten Base, beispielsweise Alkalimetallhydroxid.

Im Rahmen der vorliegenden Erfindung bevorzugt sind organisch P-substituierte Phosphonate, die eine Phosphor-Kohlenstoff-Bindung aufweisen (Phosphor-organische Verbindungen). Ihre allgemeine Struktur ist R1P(O)(OR2)₂, mit R1 und/oder R2 = Alkyl, Aryl oder H, wobei die Alkyl- bzw. Arylreste weitere Substitutionen aufweisen oder weitere chemische Gruppen tragen können. Organisch P-substituierte Phosphonate entstehen beispielsweise durch Michaelis-Arbusov- Reaktion. Viele dieser Phosphonate sind löslich in Wasser. Einige technisch wichtige Phosphonate tragen ferner Amino-Gruppe(n) in der Art NR-(CH2)x-PO(OH)₂ (R = Alkyl, Aryl oder H). Einige diese Aminophosphonate haben strukturelle Ähnlichkeiten mit Komplexbildnern wie EDTA, NTA oder DTPA und haben eine ähnliche Funktion.

Zu besonders bevorzugten Phosphonaten im Rahmen der vorliegenden Erfindung zahlen insbesondere Organophosphonate wie beispielsweise 1-Hydroxyethan-1,1-diphosphonsaure (HEDP), Aminotri(methylenphosphonsaure) (ATMP, auch bezeichnet als Aminotris(methylenphosphonsaure) bzw. Nitrolotris(methylenphosphonsaure) (NTMP)), Diethylentriamin- penta(methylenphosphonsaure) (DTPMP oder DETPMP oder DTPNT), Ethylendiamintetra(methylenphosphonsaure) (EDTMP, auch bezeichnet als Ethylendiamintetra(methylenphosphonsaure) sowie 2-Phosphonobutan-1,2,4-tricarbonsäure (PBS-AM, auch bezeichnet als 2-Phosphonobutan-1,2,4-tricarbonsäure bzw. 3-Carboxy-3-phosphonoadipinsäure), die zumeist in Form ihrer Ammonium- oder Alkalimetallsalze eingesetzt werden. Besonders bevorzugt sind Diethylentriaminpenta(methylenphosphonsäure)-Natrium, insbesondere für Waschmittel im Sinne der Erfindung, und/oder 1-Hydroxyethan-1,1-diphosphonsäure (HEDP), insbesondere für erfindungsgemäße Geschirrspülmittel und hierunter insbesondere maschinelle Geschirrspülmittel. Derartige Phosphonate sind beispielsweise unter den Handelsnamen Dequest® 2066 und Dequest® 2010 jeweils Firma Thermphos) erhältlich.

In einer bevorzugten Ausführungsform der Erfindung ist das Wasch- oder Reinigungsmittel dadurch gekennzeichnet, dass das Phosphonat in einer Menge von 0,01 bis 4 Gew.-% enthalten ist. Weitere bevorzugte Mengen des in dem Wasch- oder Reinigungsmittel enthaltenen Phosphonats sind von 0,01 bis 3 Gew.-%, von 0,01 bis 2,5 Gew.-%, von 0,02 bis 2,4 Gew.-%, von 0,02 bis 2 Gew.-%, von 0,03 bis 1,5 Gew.-% oder von 0,05 bis 1 Gew.-%.

In einer Ausführungsform der Erfindung ist die Protease in einem Wasch- oder Reinigungsmittel vorzugsweise in einer Menge von 1 x 10⁻⁸ bis 5 Gewichts-Prozent enthalten bezogen auf aktives Protein. Zunehmend bevorzugt ist die Protease in dem Mittel in einer Menge von 0,001 bis 5 Gew.-%, weiter bevorzugt von 0,01 bis 5 Gew.-%, noch weiter bevorzugt von 0,05 bis 4 Gew.-% und besonders bevorzugt von 0,075 bis 3,5 Gew.-% enthalten. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A.G. Gornall, C. S. Bardawill und M.M. David, J. Biol. Chem., 177 (1948), S. 751-766) bestimmt werden.

In einer weiteren Ausführungsform der Erfindung ist das Wasch- oder Reinigungsmittel dadurch gekennzeichnet, dass es ferner eine Komponente umfasst, die ausgewählt ist aus
i. anionische und/oder polyanionische Substanz, und/oder
ii. kationische und/oder polykationische Substanz, und/oder
iii. Hydroxyl- und/oder Polyhydroxyl-Gruppe(n) aufweisende Substanz.

Es wurde festgestellt, dass der Zusatz solcher Substanzen die Reinigungsleistung von Wasch- und Reinigungsmitteln, insbesondere von flüssigen Wasch- oder Reinigungsmitteln, welche Proteasen enthalten, insbesondere solche wie vorstehend beschrieben, enthalten, weiter verbessert, insbesondere bei vergleichsweise niedrigen Temperaturen, insbesondere zwischen 10°C und 50°C, zwischen 10°C und 40°C, zwischen 10°C und 30°C und/oder zwischen 20°C und 40°C.

Bei den vorstehend unter i. angegebenen Substanzen handelt es sich um anionische oder polyanionische Substanzen, d.h. diese Substanzen tragen mindestens eine und bevorzugt mehrere negative Ladungen. Bevorzugt handelt es sich um ein Polymer mit mindestens einem negativ geladenen Monomer, bevorzugt mit mehreren negativ geladenen Monomeren. Erfindungsgemäß bevorzugt ist dieses Polymer daher ein negativ geladenes Polymer. Bevorzugt sind beispielsweise Polymere organischer Sauren bzw. deren Salze, insbesondere Polyacrylate und/oder Poly-Zuckersäuren und/oder Polyarcylat-copolymere und/oder Polyzucker-copolymere. Diesbezüglich weitere bevorzugte Verbindungen sind Polyacrylsulfonate oder Polycarboxylate und deren Salze, Copolymere oder Salze der Copolymere.

Beispiele für besonders bevorzugt einzusetzende Substanzen sind Acusol 587D (Polyacrylsulfonat; Unternehmen Rohm & Haas/Dow Chemical), Acusol 445N (Polycarboxylat Natriumsalz; Unternehmen Rohm & Haas/Dow Chemical), Acusol 590 (Polyarcrylatcopolymer; Unternehmen Rohm & Haas/Dow Chemical), Acusol 916 (Polyarcrylat Natriumsalz; Unternehmen Rohm & Haas/Dow Chemical), Sokalan CP42 (modifiziertes Polycarboxylat Natriumsalz; Unternehmen BASF), Sokalan PA 30CL (Polycarboxylat Natriumsalz; Unternehmen BASF), Dequest P 9000 (Polymaleinsäure; Unternehmen Thermphos), Alginsäure, Poly-2-acrylamido-2-methyl-1-propan-sulfonsäure, Poly-4-styrol sulfonsäure -co-maleinsäure Natriumsalz, Poly-acrylamido-co-acrylsäure Natriumsalz, Polymethacrylsäure Natriumsalz, Poly-methyl vinyl ether-alt maleinsäure oder Polyvinylsulfonsäure Natriumsalz.

Bei den unter ii. angegebenen Substanzen handelt es sich um kationische oder polykationische Substanzen, d.h. diese Substanzen tragen mindestens eine und bevorzugt mehrere positive Ladungen. Bevorzugt handelt es sich um ein Polymer mit mindestens einem positiv geladenen Monomer, bevorzugt mit mehreren positiv geladenen Monomeren. Erfindungsgemäß bevorzugt ist dieses Polymer daher ein positiv geladenes Polymer. Beispiele für diesbezüglich bevorzugte Verbindungen sind Salze der Polyamine, Polyethyleneimine bzw. deren Copolymere, Salze der Polyallylamine, Salze der Polydiallyldimethylammonium-verbindungen oder Poly(acrylamide-co-diallyldimethylammonium-verbindungen.

Bei den unter iii. angegebenen Substanzen handelt es sich um Substanzen, die mindestens eine Hydroxyl- und/oder Polyhydroxyl-Gruppe und bevorzugt mehrere Hydroxyl- und/oder Polyhydroxyl- Gruppen aufweisen. Bevorzugt sind diesbezüglich beispielsweise Polyvinylalkohole, beispielsweise solche, die unter dem Handelsnamen Mowiol verfügbar sind (Unternehmen Kremer Pigmente GmbH & Co. KG).

Es wird an dieser Stelle ausdrücklich darauf hingewiesen, dass eine konkrete Substanz zu einer oder mehreren der vorstehend genannten Gruppen i. bis iii. zugehörig sein kann. Beispielsweise kann es sich um ein anionisches Polymer handeln, welches eine oder mehrere Hydroxyl- und/oder Polyhydroxyl-Gruppe(n) aufweist. Eine solche Substanz ist dann zugehörig zu den Gruppen i. und iii. Ebenso ist ein kationisches Polymer, welches eine oder mehrere Hydroxyl- und/oder Polyhydroxyl-Gruppe(n) aufweist, zugehörig zu den Gruppen ii. und iii.

Im Rahmen der vorliegenden Erfindung ebenfalls einsetzbar sind Derivate der vorstehend als zugehörig zu i., ii. oder iii. genannten Substanzen. Unter einem Derivat wird im Sinne der vorliegenden Anmeldung eine solche Substanz verstanden, die ausgehend von einer der vorstehend genannten Substanzen chemisch modifiziert ist, beispielsweise durch die Umwandlung einer Seitenkette oder durch kovalente Bindung einer anderen Verbindung an die Substanz. Bei solch einer Verbindung kann es sich beispielsweise um niedrigmolekulare Verbindungen wie Lipide oder Mono-, Oligo- oder Polysaccharide oder Amine bzw. Aminverbindungen handeln. Ferner kann die Substanz glykosyliert, hydrolysiert, oxidiert, N-methyliert, N-formyliert, N-acetyliert sein oder Methyl, Formyl, Ethyl, Acetyl, t-Butyl, Anisyl, Benzyl, Trifluroacetyl, N-hydroxysuccinimide, t- Butyloxycarbonyl, Benzoyl, 4-Methylbenzyl, Thioanizyl, Thiocresyl, Benzyloxymethyl, 4-Nitrophenyl, Benzyloxycarbonyl, 2-Nitrobenzoyl, 2-Nitrophenylsulphenyl, 4-Toluenesulphonyl, Pentafluorophenyl, Diphenylmethyl, 2-Chlorobenzyloxycarbonyl, 2,4,5-trichlorophenyl, 2- bromobenzyloxycarbonyl, 9-Fluorenylmethyloxycarbonyl, Tripheylmethyl, 2,2,5,7,8-pentamethyl-chroman-6-sulphonyl enthalten. Ebenso ist unter einem Derivat die kovalente oder nichtkovalente Bindung der Substanz an einen makromolekularen Träger zu verstehen, genauso wie auch ein nichtkovalenter Einschluss in geeignete makromolekulare Käfigstrukturen. Auch Kopplungen mit sonstigen makromolekularen Verbindungen, wie etwa Polyethylenglykol, können vorgenommen sein. Weitere bevorzugte chemische Modifikationen sind die Modifikation von einer oder mehreren der chemischen Gruppen -COOH, -OH, =NH, -NH₂ -SH zu -COOR, -OR, -NHR, -NR2, -NHR, -NR, -SR; wobei:
R ist-CH=CH-R2, -C=C-R2, -C(R2)=CH2, -C(R2)=C(R3), -CH=NR2, -C(R2)=N-R3, ein 4-7 C-Ring System mit oder ohne Substitution, ein 4-7 Stickstoffheterozyklus mit oder ohne Substitution, oder eine C₂ bis C₈ Kette mit 1 bis 5 Doppel- oder Dreifachbindungen mit Substitutionen ausgewählt aus R1, R2, oder R3, wobei
   - R1 ist H, -R, - NO₂, -CN, Halogenidsubstituent, -N3, -C1-8 alkyl, -(CH₂)nCO₂R2, -C2-8 alkenyl-CO₂R2, -O(CH₂)nCO₂R2, -C(O)NR2R3, -P(O)(OR2)₂, alkyl substituiertes tetrazol-5-yl, -(CH₂)nO(CH₂)n aryl, -NR2R3, -(CH₂)nOR2, -(CH₂)nSR2, -N(R2)C(O)R3,-S(O₂)NR2R3, -N(R2)S(O₂)R3, -(CHR2)n NR2R3, -C(O)R3, (CH₂)n N(R3)C(O)R3,-N(R2)CR2R3, substituiertes oder nicht substituiertes (CH2)n- zykloalkyl, substituiertes oder nicht substituiertes (CH₂)n-phenyl, oder -zyklus; wobei n eine Zahl größer als 1 ist;
   - R2 ist H, Halogenidsubstituent, -alkyl, -halogenalkyl, -(CH₂)n-phenyl,-(CH₂)1-3-biphenyl, -(CH₂)1-4-Ph-N(SO₂-C1-2-alkyl)₂, -CO(CHR1)n-OR1, -(CHR1)n-Heterozyklus,-(CHR1)n-NH-CO-R1, -(CHR1)n-NH-SO₂R1, -(CHR1)n-Ph-N(SO₂-C1-2-alkyl)₂,-(CHR1)n-C(O)(CHR1)-NHR1, -(CHR1)n-C(S)(CHR1)-NHR1, -(CH₂)nO(CH₂)nCH3,-CF3, -C₂-C₅ acyl, -(CHR1)nOH, -(CHR1)nCO₂R1, -(CHR1)n-O-alkyl, -(CHR1)n-O-(CH₂)n-O-alkyl, -(CHR1)n-S-alkyl, -(CHR1)n-S(O)-alkyl, -(CHR1)n-S(O₂)-alkyl, (CHR1)n-S(O₂)-NHR3,-(CHR3)n-N3, -(CHR3)nNHR4, eine C₂ bis C₈ Alken-Kette mit 1 bis 5 Doppelbindungen, eine C₂ bis C₈ Alkin-Kette mit 1 bis 5 Dreifachbindungen, substituierter oder nicht substituierter -(CHR3)n Heterozyklus, substituiertes oder nicht substituiertes gesättigtes oder nicht gesättigtes -(CHR3)n Zykloalkyl; wobei n eine Zahl größer als 1 ist und R1 und R3 gleich oder unterschiedlich sein können;
   - R3 ist H, -OH, -CN, substituiertes Alkyl, - C₂ bis C₈ Alkenyl, substituiertes oder nicht substituiertes Zykloalkyl, -N(R1)R2, gesättigter oder nicht gesättigter C₅ bis C₇ Heterozyklus oder Heterobizyklus von 4 bis 7 C-Atomen, -NR 1, -NR2, -NR1 R2 bestehend aus einem gesättigten oder nicht gesättigten Heterozyklus oder einem Heterobizyklus von 4 bis 7 C-Atomen;
   - R4 ist H, -(CH₂)nOH, -C(O)OR5, -C(O)SR5, -(CH₂)nC(O)NR6R7, -O-C(O)-O-R6, eine Aminosäure oder ein Peptid; wobei n eine Zahl zwischen 0 und 4 ist;
   - R5 ist H;
   - R6 ist -C(R7)-(CH₂)n-O-C(O)-R8, -(CH₂)n-C(R7)-O-C(O)R8,-(CH₂)n-C(R7)-O-C(O)-O-R8, oder -C(R7)-(CH₂)n-O-C(O)-O-R8; wobei n eine Zahl zwischen 0 und 4 ist; und
   - R7 und R8 sind jeweils H, Alkyl, substituiertes Alkyl, Aryl, substituiertes Aryl, Alkenyl, substituiertes Alkenyl, Alkinyl, substituiertes Alkinyl, Heterozyklus, substituierter Heterozyklus, Alkylaryl, substituiertes Alkylaryl, Zykloalkyl, substituiertes Zykloalkyl, oder CH₂CO₂ alkyl, wobei R7 und R8 gleich oder unterschiedlich sein können.

Erfindungsgemäß ist es weiter möglich, alle möglichen Kombinationen der vorstehend als zugehörig zu i., ii. oder iii. genannten Substanzen und/oder deren Derivate einzusetzen.

In einer bevorzugten Ausführungsform der Erfindung kann dasflüssig Wasch- oder Reinigungsmittel als solches oder nach Verdünnen mit Wasser zur Reinigung von Textilien und/oder harten Oberflächen eingesetzt werden. Eine solche Verdünnung kann leicht hergestellt werden, indem eine abgemessene Menge des Mittels in einer weiteren Menge Wasser verdünnt wird in bestimmten Gewichtsverhältnissen von Mittel : Wasser und optional diese Verdünnung geschüttelt wird, um eine gleichmäßige Verteilung des Mittels im Wasser sicherzustellen. Mögliche Gewichts- oder Volumenverhältnisse der Verdünnungen sind von 1:0 Mittel : Wasser bis 1:10000 oder 1:20000 Mittel : Wasser, vorzugsweise von 1:10 bis 1:2000 Mittel : Wasser.

Als flüssige Wasch- oder Reinigungsmittel können hierbei alle flüssigen bzw. fließfähigen Darreichungsformen dienen. "Fließfähig" im Sinne der vorliegenden Anmeldung sind dabei Mittel, welche gießbar sind und Viskositäten bis hin zu mehreren 10.000 mPas aufweisen können. Die Viskosität kann mit üblichen Standardmethoden (beispielsweise Brookfield-Viskosimeter LVT-11 bei 20 U/min und 20°C, Spindel 3) gemessen werden und liegt vorzugsweise im Bereich von 5 bis 10000 mPas. Bevorzugte Mittel haben Viskositäten von 10 bis 8000 mPas, wobei Werte zwischen 120 bis 3000 mPas besonders bevorzugt sind. Ein flüssiges Wasch- oder Reinigungsmittel im Rahmen der vorliegenden Erfindung kann daher auch gelförmig oder pastenförmig sein, es kann als homogene Lösungen oder Suspensionen vorliegen, sowie beispielsweise versprühbar oder in sonstigen üblichen Darreichungsformen konfektioniert sein. Zu den Waschmitteln zählen alle denkbaren Waschmittelarten, insbesondere Waschmittel für Textilien, Teppiche oder Naturfasern. Sie können für die manuelle und/oder auch die maschinelle Anwendung vorgesehen sein. Zu den Waschmitteln zählen ferner Waschhilfsmittel, die bei der manuellen oder maschinellen Textilwasche zum eigentlichen Waschmittel hinzudosiert werden, um eine weitere Wirkung zu erzielen. Zu den Reinigungsmitteln werden alle, ebenfalls in sämtlichen genannten Darreichungsformen vorkommenden Mittel zur Reinigung und/oder Desinfektion harter Oberflächen, manuelle und maschinelle Geschirrspülmittel, Teppichreiniger, Scheuermittel, Glasreiniger, WC-Duftspüler, usw. gezählt. Textilvor- und Nachbehandlungsmittel sind schließlich auf der einen Seite solche Mittel, mit denen das Waschestück vor der eigentlichen Wäsche in Kontakt gebracht wird, beispielsweise zum Anlösen hartnäckiger Verschmutzungen, auf der anderen Seite solche, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet. Desinfektionsmittel sind beispielsweise Handedesinfektionsmittel, Flächendesinfektionsmittel und Instrumentendesinfektionsmittel, die ebenfalls in den genannten Darreichungsformen vorkommen können.

In einer weiteren bevorzugten Ausführungsform der Erfindung ist das Wasch- oder Reinigungsmittel dadurch gekennzeichnet, dass es mindestens einen weiteren Inhaltsstoff umfasst, insbesondere einen, der ausgewählt ist aus der Gruppe bestehend aus Tensid, Gerüststoff (Builder), Persauerstoffverbindung, Bleichaktivator, Alkohol, Säure, Vergrauungsinhibitor, optischer Aufheller, Schauminhibitor, wasserlösliches Salz, Verdickungsmittel, flüchtiges Alkali und/oder Base, hydrophilierendes Agens sowie Kombinationen hiervon.

Als Tensid(e) können anionische, nichtionische, zwitterionische und/oder amphotere Tenside eingesetzt werden. Bevorzugt sind aus anwendungstechnischer Sicht Mischungen aus anionischen und nichtionischen Tensiden. Der Gesamttensidgehalt des flüssigen Wasch- oder Reinigungsmittels liegt vorzugsweise unterhalb von 60 Gew.-% und besonders bevorzugt unterhalb von 45 Gew.-%, bezogen auf das gesamte flüssige Wasch- oder Reinigungsmittel.

Geeignete nichtionische Tenside umfassen alkoxylierte Fettalkohole, alkoxylierte Fettsäurealkylester, Fettsäureamide, alkoxylierte Fettsäureamide, Polyhydroxyfettsäureamide, Alkylphenolpolyglycolether, Aminoxide, Alkylpolyglucoside und Mischungen daraus.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄ Alkohole mit 3 EO, 4 EO oder 7 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Geeignet sind ferner auch eine Mischung aus einem (starker) verzweigten ethoxylierten Fettalkohol und einem unverzweigten ethoxylierten Fettalkohol, wie beispielsweise eine Mischung aus einem C₁₆₋₁₃-Fettalkohol mit 7 EO und 2-Propylheptanol mit 7 EO. Insbesondere bevorzugt enthält das Wasch-, Reinigungs-, Nachbehandlungs- oder Waschhilfsmittel einen C₁₂₋₁₈-Fettalkohol mit 7 EO oder einen C₁₃₋₁₅-Oxoalkohol mit 7 EO als nichtionisches Tensid.

Der Gehalt an nichtionischen Tensiden beträgt in dem Wasch- oder Reinigungsmittel bevorzugt 3 bis 40 Gew.-%, vorzugsweise 5 bis 30 Gew.-% und insbesondere 7 bis 20 Gew.-%, jeweils bezogen auf das gesamte Wasch- oder Reinigungsmittel.

Neben den nichtionischen Tensiden kann das Wasch- oder Reinigungsmittel auch anionische Tenside enthalten. Als anionisches Tensid werden vorzugsweise Sulfonate, Sulfate, Seifen, Alkylphosphate, anionische Silikontenside und Mischungen daraus eingesetzt.

Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈- Monoolefinenmit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch C₁₂₋₁₈- Alkansulfonate und die Ester von α-Sulfofettsäuren (Estersulfonate), zum Beispiel die α-sulfonierten Methylester der hydrierten Kokos-, Palmkern- oder Talgfettsäuren.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Aus waschtechnischem Interesse sind die C₁₂-C₁₆- Alkylsulfate und C₁₂-C₁₅- Alkylsulfate sowie C₁₄-C₁₅- Alkylsulfate bevorzugt. Auch 2,3-Alkylsulfate sind geeignete anionische Tenside.

Auch die Schwefelsäuremonoester der mit 1 bis 6 Mol Ethylenoxid ethoxylierten geradkettigen oder verzweigten C₇₋₂₁- Alkohole, wie 2-Methyl-verzweigte C₉₋₁₁- Alkohole mit im Durchschnitt 3,5 Mol Ethylenoxid (EO) oder C₁₂₋₁₈-Fettalkohole mit 1 bis 4 EO, sind geeignet.

Auch bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside einschließlich der Seifen können in Form ihrer Natrium-, Kalium- oder Magnesium- oder Ammoniumsalze vorliegen. Vorzugsweise liegen die anionischen Tenside in Form ihrer Natriumsalze vor. Weitere bevorzugte Gegenionen für die anionischen Tenside sind auch die protonierten Formen von Cholin, Triethylamin oder Methylethylamin.

Der Gehalt eines Wasch- oder Reinigungsmittels an anionischen Tensiden kann 1 bis 40 Gew.-%, vorzugsweise 5 bis 30 Gew.-% und ganz besonders bevorzugt 10 bis 25 Gew.-%, jeweils bezogen auf das gesamte Wasch- oder Reinigungsmittel, betragen.

Als Gerüststoffe, die in dem Wasch- oder Reinigungsmittel enthalten sein können, sind insbesondere Silikate, Aluminiumsilikate (insbesondere Zeolithe), Carbonate, Salze organischer Di- und Polycarbonsäuren sowie Mischungen dieser Stoffe zu nennen.

Organische Gerüststoffe, welche in dem Wasch- oder Reinigungsmittel vorhanden sein können, sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), Methylglycindiessigsäure (MGDA) und deren Abkömmlinge sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen.

Als Gerüststoffe sind weiter polymere Polycarboxylate geeignet. Dies sind beispielsweise die Alkalimetallsalze der Polyacrylsäure oder der Polymethacrylsäure, zum Beispiel solche mit einer relativen Molekülmasse von 600 bis 750.000 g/mol.

Geeignete Polymere sind insbesondere Polyacrylate, die bevorzugt eine Molekülmasse von 1.000 bis 15.000 g/mol aufweisen. Aufgrund ihrer überlegenen Löslichkeit können aus dieser Gruppe wiederum die kurzkettigen Polyacrylate, die Molmassen von 1.000 bis 10.000 g/mol, und besonders bevorzugt von 1.000 bis 5.000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Zur Verbesserung der Wasserlöslichkeit können die Polymere auch Allylsulfonsäuren, wie Allyloxybenzolsulfonsäure und Methallylsulfonsäure, als Monomer enthalten.

Bevorzugt werden allerdings lösliche Gerüststoffe, wie beispielsweise Citronensäure, oder Acrylpolymere mit einer Molmasse von 1.000 bis 5.000 g/mol bevorzugt in den flüssigen Wasch- oder Reinigungsmitteln eingesetzt.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, Mitteln eingesetzt.

Als für den Einsatz in Mitteln im Sinne der Erfindung geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Waschbedingungen Wasserstoffperoxid abgebende an- organische Salze, zu denen Perborat, Percarbonat, Persilikat und/oder Persulfat wie Caroat gehören, in Betracht. Falls ein Mittel Persauerstoffverbindungen enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphonaten, Boraten beziehungsweise Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5- Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofüran und Enolester sowie acetyliertes Sorbitol und Mannitol beziehungsweise deren beschriebene Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam. Die hydrophil substituierten Acylacetale und die Acyllactame werden ebenfalls bevorzugt eingesetzt. Auch Kombinationen konventioneller Bleichaktivatoren können eingesetzt werden. Derartige Bleichaktivatoren können, insbesondere bei Anwesenheit obengenannter Wasserstoffperoxid-liefernder Bleichmittel, im üblichen Mengenbereich, vorzugsweise in Mengen von 0,5 Gew.-% bis 10 Gew.-%, insbesondere 1 Gew.-% bis 8 Gew.-%, bezogen auf das gesamte Mittel, enthalten sein, fehlen bei Einsatz von Percarbonsäure als alleinigem Bleichmittel jedoch vorzugsweise ganz.

Zusätzlich zu den konventionellen Bleichaktivatoren oder an deren Stelle können auch Sulfonimine und/oder bleichverstärkende Übergangsmetallsalze beziehungsweise Übergangsmetallkomplexe als sogenannte Bleichkatalysatoren enthalten sein.

Die Wasch- oder Reinigungsmittel sind flüssig und enthalten vorzugsweise Wasser als Hauptlösungsmittel. Daneben können dem Wasch- oder Reinigungsmittel nichtwässrige Lösungsmittel zugesetzt werden. Geeignete nichtwässrige Lösungsmittel umfassen ein- oder mehrwertige Alkohole, Alkanolamine oder Glykolether, sofern sie im angegebenen Konzentrationsbereich mit Wasser mischbar sind. Vorzugsweise werden die Lösungsmittel ausgewählt aus Ethanol, n-Propanol, i-Propanol, Butanolen, Glykol, Propandiol, Butandiol, Glycerin, Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropylenglykolmonoethylether, Di-isopropylenglykolmonomethylether, Di-isopropylenglykolmonoethylether, Methoxytriglykol, Ethoxytriglykol, Butoxytriglykol, 1-Butoxyethoxy-2-propanol, 3-Methyl-3-methoxybutanol, Propylen-glykol-t-butylether, Di-n-octylether sowie Mischungen dieser Lösungsmittel. Es ist allerdings bevorzugt, dass das Wasch- oder Reinigungsmittel ein Polyol als nicht-wässriges Lösungsmittel enthält. Das Polyol kann insbesondere Glycerin, 1,2-Propandiol, 1,3-Propandiol, Ethylenglycol, Diethylenglycol und/oder Dipropylenglycol umfassen. Insbesondere bevorzugt enthält das Wasch- oder Reinigungsmittel eine Mischung aus einem Polyol und einem einwertigen Alkohol. Nichtwässrige Lösungsmittel können in dem Wasch- oder Reinigungsmittel in Mengen zwischen 0,5 und 15 Gew.-%, bevorzugt aber unter 12 Gew.-% und eingesetzt werden.

Zur Einstellung eines gewünschten, sich durch die Mischung der übrigen Komponenten nicht von selbst ergebenden pH-Werts können die Mittel system- und umweltvertragliche Sauren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den Mitteln in Mengen von vorzugsweise nicht über 20 Gew.-%, insbesondere von 1,2 Gew.-% bis 17 Gew.-%, enthalten.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt werden Celluloseether, wie Carboxymethylcellulose (Na-Salz), Methylcellulose, Hydroxyalkylcellulose und Mischether, wie Methylhydroxyethylcellulose, Methyl-hydroxypropylcellulose, Methylcarboxymethylcellulose und deren Gemische, beispielsweise in Mengen von 0,1 bis 5 Gew.-%, bezogen auf das Mittel, eingesetzt.

Textilwaschmittel können als optische Aufheller beispielsweise Derivate der Diaminostilbendisulfonsäure beziehungsweise deren Alkalimetallsalze enthalten, obgleich sie für den Einsatz als Colorwaschmittel vorzugsweise frei von optischen Aufhellern sind. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten optischen Aufheller können verwendet werden.

Insbesondere beim Einsatz in maschinellen Verfahren kann es von Vorteil sein, den Mitteln Schauminhibitoren zuzusetzen. Als Schauminhibitoren eignen sich beispielsweise Seifen natürlicher oder synthetischer Herkunft, die einen hohen Anteil an C₁₈-C₂₄-Fettsauren aufweisen. Geeignete nichttensidartige Schauminhibitoren sind beispielsweise Organopolysiloxane und deren Gemische mit mikrofeiner, gegebenenfalls silanierter Kieselsäure sowie Paraffine, Wachse, Mikrokristallinwachse und deren Gemische mit silanierter Kieselsäure oder Bisfettsäurealkylendiamiden. Mit Vorteilen werden auch Gemische aus verschiedenen Schauminhibitoren verwendet, zum Beispiel solche aus Silikonen, Paraffinen oder Wachsen.

Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinen und Bistearylethylendiamid bevorzugt.

Ein Mittel im Sinne der Erfindung kann weiterhin ein oder mehrere wasserlösliche Salze enthalten, die beispielsweise zur Viskositätseinstellung dienen. Es kann sich dabei um anorganische und/oder organische Salze handeln. Einsetzbare anorganische Salze sind dabei vorzugsweise ausgewählt aus der Gruppe umfassend farblose wasserlösliche Halogenide, Sulfate, Sulfite, Carbonate, Hydrogencarbonate, Nitrate, Nitrite, Phosphate und/oder Oxide der Alkalimetalle, der Erdalkalimetalle, des Aluminiums und/oder der Übergangsmetalle; weiterhin sind Ammoniumsalze einsetzbar. Besonders bevorzugt sind dabei Halogenide und Sulfate der Alkalimetalle; vorzugsweise ist das anorganische Salz daher ausgewählt aus der Gruppe umfassend Natriumchlorid, Kaliumchlorid, Natriumsulfat, Kaliumsulfat sowie Gemische derselben. Einsetzbare organische Salze sind beispielsweise farblose wasserlösliche Alkalimetall-, Erdalkalimetall-, Ammonium-, Aluminium- und/oder Übergangsmetallsalze der Carbonsäuren. Vorzugsweise sind die Salze ausgewählt aus der Gruppe umfassend Formiat, Acetat, Propionat, Citrat, Malat, Tartrat, Succinat, Malonat, Oxalat, Lactat sowie Gemische derselben.

Ein Mittel im Sinne der Erfindung kann weiterhin zur Verdickung ein oder mehrere Verdickungsmittel enthalten. Bevorzugt ist das Verdickungsmittel ausgewählt aus der Gruppe umfassend Xanthan, Guar, Carrageenan, Agar-Agar, Gellan, Pektin, Johannisbrotkernmehl und Mischungen daraus. Diese Verbindungen sind auch in Gegenwart von anorganischen Salzen effektive Verdickungsmittel. In einer besonders bevorzugten Ausführungsform enthält das Wasch- oder Reinigungsmittel Xanthan als Verdickungsmittel, da Xanthan auch in Gegenwart von hohen Salzkonzentrationen effektiv verdickt und eine makroskopische Auftrennung der kontinuierlichen Phase verhindert. Zusätzlich stabilisiert das Verdickungsmittel die kontinuierliche, tensidarme Phase und verhindert eine makroskopische Phasenseparation.

Alternativ oder ergänzend können auch (Meth)Acrylsäure(co)polymere als Verdickungsmittel eingesetzt werden. Geeignete Acryl- und Methacryl(co)polymere umfassen beispielsweise die hochmolekularen mit einem Polyalkenylpolyether, insbesondere einem Allylether von Saccharose, Pentaerythrit oder Propylen, vernetzten Homopolymere der Acrylsaure (INCI-Bezeichnung gemäß "International Dictionary of Cosmetic Ingredients" der "The Cosmetic, Toiletry and Fragrance Association (CTFA)": Carbomer), die auch als Carboxyvinylpolymere bezeichnet werden. Solche Polyacrylsäuren sind unter anderem unter den Handelsnamen Polygel® und Carbopol® erhältlich. Weiterhin sind beispielsweise folgende Acrylsäure-Copolymere geeignet: (i) Copolymere von zwei oder mehr Monomeren aus der Gruppe der Acrylsäure, Methacrylsäure und ihrer einfachen, vorzugsweise mit C₁₋₄-Alkanolen gebildeten, Ester (INCi Acrylates Copolymer), die beispielsweise unter den Handelsnamen Aculyn®, Acusol® oder Tega® Polymer erhältlich sind; (ii) vernetzte hochmolekulare Acrylsäure-Copolymere, zu denen etwa die mit einem Allylether der Saccharose oder des Pentaerythrits vernetzten Copolymere von C₁₀₋₃₀-Alkylacrylaten mit einem oder mehreren Monomeren aus der Gruppe der Acrylsäure, Methacrylsäure und ihrer einfachen, vorzugsweise mit C₁₋₄-Alkanolen gebildeten, Ester (INCi Acrylates/C₁₀₋₃₀ Alkyl Acrylate Crosspolymer) gehören und die beispielsweise unter dem Handelsnamen Carbopol® erhältlich sind. Weitere geeignete Polymere sind (Meth)Acrylsäure(co)polymere des Typs Sokalan®.

Es kann bevorzugt sein, dass das Wasch- oder Reinigungsmittel ein (Meth)Acrylsäure(co)polymer in Kombination mit einem weiteren Verdickungsmittel, vorzugsweise Xanthan, enthält. Das Wasch- oder Reinigungsmittel kann 0,05 bis 1,5 Gew.-% und vorzugsweise 0,1 bis 1 Gew.-%, jeweils bezogen auf das gesamte Wasch- oder Reinigungsmittel, Verdickungsmittel enthalten. Die Menge an eingesetztem Verdickungsmittel ist dabei abhängig von der Art des Verdickungsmittels und dem gewünschten Grad der Verdickung.

Ein entsprechendes Mittel kann weiterhin flüchtiges Alkali enthalten. Als solches werden Ammoniak und/oder Alkanolamine, die bis zu 9 C Atome im Molekül enthalten können, verwendet.

Als Alkanolamine werden die Ethanolamine bevorzugt und von diesen wiederum das Monoethanolamin. Der Gehalt an Ammoniak und/oder Alkanolamin beträgt vorzugsweise 0,01 bis 2 Gew.-%; besonders bevorzugt wird Ammoniak eingesetzt. Daneben können auch geringe Mengen an Basen enthalten sein. Bevorzugte Basen stammen aus der Gruppe der Alkali- und Erdalkalimetallhydroxide und -carbonate, insbesondere der Alkalimetallhydroxide, von denen Kaliumhydroxid und vor allem Natriumhydroxid besonders bevorzugt ist.

Ein entsprechendes Mittel kann auch ein hydrophilierendes Agens enthalten. Hierunter werden im Rahmen der vorliegenden Erfindung Mittel zur Hydrophilierung von Oberflächen verstanden. Zur Hydrophilierung eignen sich insbesondere kolloidale Silica-Sole, in denen das Siliciumdioxid vorzugsweise nanopartikular vorliegt. Kolloidale nanopartikulare Silica-Sole im Sinne dieser Erfindung sind stabile Dispersionen von amorphem partikularem Siliciumdioxid SiO₂ mit Partikelgrößen im Bereich von 1 bis 100 nm. Vorzugsweise liegen die Teilchengrößen dabei im Bereich 3 bis 50 nm, besonders bevorzugt 4 bis 40 nm. Ein Beispiel für ein Silica-Sol, welches geeignet ist, im Sinne dieser Erfindung eingesetzt zu werden, ist das unter dem Handelsnamen Bindzil® 30/360 von der Firma Akzo erhältliche Silica-Sol mit einer Partikelgröße von 9 nm.

Weitere geeignete Silica-Sole sind Bindzil® 15/500, 30/220, 40/200 (Akzo), Nyacol® 215, 830, 1430, 2034DI sowie Nyacol® DP5820, DP5480, DP5540 etc. (Nyacol Products), Levasil® 100/30, 100F/30, 100S/30, 200/30, 200F/30, 300F/30, VP 4038, VP 4055 (H.C. Starck/ Bayer) oder auch CAB-O-SPERSE® PG 001, PG 002 (wässrige Dispersionen von CAB-O-SIL®, Cabot), Quartron PL-1, PL-3 (FusoChemical Co.), Kostrosol 0830, 1030, 1430 (Chemiewerk Bad Köstritz). Bei den eingesetzten Silica-Solen kann es sich auch um oberflächenmodifiziertes Silica handeln, das mit Natriumaluminat behandelt wurde (Aluminamodifiziertes Silica).

Daneben lassen sich auch bestimmte Polymere zur Hydrophilierung von Oberflächen einsetzen. Als hydrophilierende Polymere sind insbesondere amphotere Polymer geeignet, beispielsweise Copolymere aus Acryl- oder Methacrylsäure und MAPTAC, DADMAC oder einer anderen polymerisierbaren quaternären Ammoniumverbindung. Weiterhin können auch Copolymere mit AMPS (2-Acrylamido-2-methylpropansulfonsaure) verwendet werden. Polyethersiloxane, also Copolymere von Polymethylsiloxanen mit Ethylenoxid- oder Propylenoxidsegmenten sind weitere geeignete Polymere. Ebenfalls einsetzbar sind Acrylpolymere, Maleinsäure-Copolymere und Polyurethane mit PEG (Polyethylenglykol)-Einheiten. Geeignete Polymere sind beispielsweise unter den Handelsnamen Mirapol Surf-S 100, 110, 200, 210, 400, 410, A 300, A 400 (Rhodia), Tegopren 5843 (Goldschmidt), Sokalan CP 9 (BASF) oder Polyquart Ampho 149 (Cognis) kommerziell erhältlich.

Die zu wählenden Inhaltsstoffe des Mittels wie auch die Bedingungen, unter denen es erfindungsgemäß angewendet wird, wie beispielsweise Temperatur, pH-Wert, Ionenstärke, Redox-Verhältnisse oder mechanische Einflüsse, sind üblicherweise für das jeweilige Anwendungsgebiet optimiert.

Flüssige beziehungsweise pastöse Mittel in Form von übliche Lösungsmittel enthaltenden Lösungen werden in der Regel durch einfaches Mischen der Inhaltsstoffe, die in Substanz oder als Lösung in einen automatischen Mischer gegeben werden können, hergestellt.

Wasch- oder Reinigungsmittel im Sinne der Erfindung können ausschließlich eine Protease enthalten wie beschrieben. Alternativ können sie auch weitere hydrolytische Enzyme oder andere Enzyme in einer für die Wirksamkeit des Mittels zweckmäßigen Konzentration enthalten. Im Sinne der Erfindung können somit Mittel eines oder mehrere weitere Enzyme umfassen, wobei prinzipiell alle im Stand der Technik für diese Zwecke etablierten Enzyme einsetzbar sind. Als weitere Enzyme bevorzugt einsetzbar sind alle Enzyme, die in dem erfindungsgemäßen Mittel eine katalytische Aktivität entfalten können, insbesondere eine insbesondere eine Protease, Amylase, Cellulase, Hemicellulase, Mannanase, Tannase, Xylanase, Xanthanase, β- Glucosidase, Carrageenase, Perhydrolase, Oxidase, Oxidoreduktase oder eine Lipase, sowie vorzugsweise deren Gemische. Diese Enzyme sind im Prinzip natürlichen Ursprungs; ausgehend von den natürlichen Molekülen stehen für den Einsatz in Wasch- und Reinigungsmitteln verbesserte Varianten zur Verfügung, die entsprechend bevorzugt eingesetzt werden.

Mittel im Sinne der Erfindung enthalten Enzyme vorzugsweise in Gesamtmengen von 1x10⁻⁸ bis 5 Gewichts-Prozent bezogen auf aktives Protein. Bevorzugt sind die Enzyme von 0,001 bis 5 Gew.-%, weiter bevorzugt von 0,01 bis 5 Gew.-%, noch weiter bevorzugt von 0,05 bis 4 Gew.-% und besonders bevorzugt von 0,075 bis 3,5 Gew.-% in Mitteln enthalten, wobei jedes enthaltene Enzym in den genannten Mengenverhältnissen vorliegen kann. Die Enzyme können an Trägerstoffen adsorbiert und/oder in Hüllsubstanzen eingebettet sein, um sie gegen vorzeitige Inaktivierung zu schützen. Die Proteinkonzentration kann mit Hilfe bekannter Methoden, zum Beispiel dem BCA-Verfahren (Bicinchoninsaure; 2,2'-Bichinolyl-4,4'-dicarbonsaure) oder dem Biuret-Verfahren (A.G. Gornall, C. S. Bardawill und M.M. David, J. Biol. Chem., 177 (1948), S. 751-766) bestimmt werden.

Besonders bevorzugt zeigen die Enzyme synergistische Effekte hinsichtlich ihrer Wirkung gegenüber bestimmter Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solcher Synergismus vor zwischen der erfindungsgemäß enthaltenen Protease und einem weiteren Enzym eines Mittels, darunter insbesondere zwischen der genannten Protease und einer Amylase und/oder einer Mannanase und/oder einerLipase.

Synergistische Effekte können nicht nur zwischen verschiedenen Enzymen, sondern auch zwischen einem oder mehreren Enzymen und weiteren Inhaltsstoffen des Mittels auftreten.

Unter den Proteasen sind solche vom Subtilisin-Typ bevorzugt. Beispiele hierfür sind die Subtilisine BPN' und Carlsberg, die Protease PB92, die Subtilisine 147 und 309, die Alkalische Protease aus Bacillus lentus, Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase® von der Firma Novozymes A/S, Bagsvrerd, Dänemark, erhältlich.

Die Subtilisine 147 und 309 werden unter den Handelsnamen Esperase®, beziehungsweise Savinase® von der Firma Novozymes vertrieben. Von der Protease aus Bacillus lentus DSM 5483 leiten sich die unter der Bezeichnung BLAP® geführten Protease-Varianten ab. Weitere brauchbare Proteasen sind beispielsweise die unter den Handelsnamen Durazym®, Relase®, Everlase®, Nafizym®, Natalase®, Kannase® und Ovozyme® von der Firma Novozymes, die unter den Handelsnamen, Purafect®, Purafect® OxP, Purafect® Prime, Excellase® und Properase® von der Firma Genencor, das unter dem Handelsnamen Protosol® von der Firma Advanced Biochemicals Ltd., Thane, Indien, das unter dem Handelsnamen Wuxi® von der Firma Wuxi Snyder Bioproducts Ltd., China, die unter den Handelsnamen Proleather® und Protease P® von der Firma Amano Pharmaceuticals Ltd., Nagoya, Japan, und das unter der Bezeichnung Proteinase K-16 von der Firma Kao Corp., Tokyo, Japan, erhältlichen Enzyme. Besonders bevorzugt eingesetzt werden auch die Proteasen aus Bacillus gibsonii und Bacillus pumilus, die offenbart sind in den internationalen Patentanmeldungen WO 2008/086916 und WO 2007/131656.

Beispiele für erfindungsgemäß konfektionierbare Amylasen sind die α-Amylasen aus Bacillus licheniformis, aus B. amyloliquefaciens oder aus B. stearothermophilus sowie deren für den Einsatz in Wasch- oder Reinigungsmitteln verbesserte Weiterentwicklungen. Das Enzym aus B. licheniformis ist von der Firma Novozymes unter dem Namen Termamyl® und von der Firma Genencor unter dem Namen Purastar®ST erhältlich. Weiterentwicklungsprodukte dieser α-Amylase sind von der Firma Novozymes unter den Handelsnamen Duramyl® und Termamyl®ultra, von der Firma Genencor unter dem Namen Purastar®OxAm und von der Firma Daiwa Seiko Inc., Tokyo, Japan, als Keistase® erhältlich. Die α-Amylase von B. amyloliquefaciens wird von der Firma Novozymes unter dem Namen BAN® vertrieben, und abgeleitete Varianten von der α-Amylase aus B. stearothermophilus unter den Namen BSG® und Novamyl®, ebenfalls von der Firma Novozymes.

Desweiteren sind für diesen Zweck die α-Amylase aus Bacillus sp. A 7-7 (DSM 12368) und die Cyclodextrin-Glucanotransferase (CGTase) aus B. agaradherens (DSM 9948) hervorzuheben. Ferner sind die amylolytischen Enzyme einsetzbar, die dem Sequenzraum von α-Amylasen angehören, der in der internationalen Patentanmeldung WO 03/002711 A2 definiert wird, und die, die in der Anmeldung WO 03/054177 A2 beschrieben werden. Ebenso sind Fusionsprodukte der genannten Moleküle einsetzbar.

Darüber hinaus sind die unter den Handelsnamen Fungamyl® von der Firma Novozymes erhältlichen Weiterentwicklungen der α-Amylase aus Aspergillus niger und A. oryzae geeignet. Weitere einsetzbare Handelsprodukte sind beispielsweise die Amylase-LT® und Stainzyme® bzw. Stainzyme ultra® oder Stainzyme plus®, letztere ebenfalls von der Firma Novozymes. Auch durch Punktmutationen erhältliche Varianten dieser Enzyme können erfindungsgemäß eingesetzt werden.

Beispiele für erfindungsgemäß konfektionierbare Lipasen oder Cutinasen, die insbesondere wegen ihrer Triglycerid-spaltenden Aktivitäten enthalten sind, aber auch, um aus geeigneten Vorstufen in situ Persäuren zu erzeugen, sind die ursprünglich aus Humicola lanuginosa (Thermomyces lanuginosus) erhältlichen, beziehungsweise weiterentwickelten Lipasen, insbesondere solche mit dem Aminosäureaustausch D96L. Sie werden beispielsweise von der Firma Novozymes unter den Handelsnamen Lipolase®, Lipolase®Ultra, LipoPrime®, Lipozyme® und Lipex® vertrieben.

Desweiteren sind beispielsweise die Cutinasen einsetzbar, die ursprünglich aus Fusarium solani pisi und Humicola insolens isoliert worden sind. Ebenso brauchbare Lipasen sind von der Firma Amano unter den Bezeichnungen Lipase CE®, Lipase P®, Lipase B®, beziehungsweise Lipase CES®, Lipase AKG®, Bacillis sp. Lipase®, Lipase AP®, Lipase M-AP® und Lipase AML® erhältlich. Von der Firma Genencor sind beispielsweise die Lipasen beziehungsweise Cutinasen einsetzbar, deren Ausgangsenzyme ursprünglich aus Pseudomonas mendocina und Fusarium solanii isoliert worden sind. Als weitere wichtige Handelsprodukte sind die ursprünglich von der Firma Gist-Brocades vertriebenen Präparationen M1 Lipase® und Lipomax® und die von der Firma Meito Sangyo KK, Japan, unter den Namen Lipase MY-30®, Lipase OF® und Lipase PL® vertriebenen Enzyme zu erwähnen, ferner das Produkt Lumafast® von der Firma Genencor.

Wasch- oder Reinigungsmittel können im Sinne der Erfindung ferner Cellulasen enthalten, je nach Zweck als reine Enzyme, als Enzympräparationen oder in Form von Mischungen, in denen sich die einzelnen Komponenten vorteilhafterweise hinsichtlich ihrer verschiedenen Leistungsaspekte ergänzen. Zu diesen Leistungsaspekten zählen insbesondere Beiträge zur Primärwaschleistung, zur Sekundärwaschleistung des Mittels (Antiredepositionswirkung oder Vergrauungsinhibition) und Avivage (Gewebewirkung), bis hin zum Ausüben eines "stone washed"-Effekts.

Eine brauchbare pilzliche, Endoglucanase(EG)-reiche Cellulase-Präparation, beziehungsweise deren Weiterentwicklungen wird von der Firma Novozymes unter dem Handelsnamen Celluzyme® angeboten. Die ebenfalls von der Firma Novozymes erhältlichen Produkte Endolase® und Carezyme® basieren auf der 50 kD-EG, beziehungsweise der 43 kD-EG aus H. insolens DSM 1800. Weitere einsetzbare Handelsprodukte dieser Firma sind Cellusoft®, Renozyme® und Celluclean®. Weiterhin einsetzbar sind beispielsweise die 20 kD-EG aus Melanocarpus, die von der Firma AB Enzymes, Finnland, unter den Handelsnamen Ecostone® und Biotouch® erhältlich sind. Weitere Handelsprodukte der Firma AB Enzymes sind Econase® und Ecopulp®. Weitere geeignete Cellulasen sind aus Bacillus sp. CBS 670.93 und CBS 669.93, wobei die aus Bacillus sp. CBS 670.93 von der Firma Genencor unter dem Handelsnamen Puradax® erhältlich ist. Weitere Handelsprodukte der Firma Genencor sind "Genencor detergent cellulase L" und IndiAge®Neutra.

Ferner können insbesondere zur Entfernung bestimmter Problemanschmutzungen weitere Enzyme eingesetzt sein, die unter dem Begriff Hemicellulasen zusammengefasst werden. Hierzu gehören beispielsweise Mannanasen, Xanthanlyasen, Pektinlyasen (=Pektinasen), Pektinesterasen, Pektatlyasen, Xyloglucanasen (=Xylanasen), Pullulanasen und β-Glucanasen. Diesbezüglich geeignete Enzyme sind beispielsweise unter den Namen Gamanase® und Pektinex AR® von der Firma Novozymes, unter dem Namen Rohapec® B1L von der Firma AB Enzymes und unter dem Namen Pyrolase® von der Firma Diversa Corp., San Diego, CA, USA erhältlich. Die aus Bacillus subtilis gewonnene β-Glucanase ist unter dem Namen Cereflo® von der Firma Novozymes erhältlich. Erfindungsgemäß besonders bevorzugte Hemicellulasen sind Mannanasen, welche beispielsweise unter den Handelsnamen Mannaway(R) von dem Unternehmen Novozymes oder Purabrite® von dem Unternehmen Genencor vertrieben werden.

Zur Erhöhung der bleichenden Wirkung können Mittel m Sinne der Erfindung auch Oxidoreduktasen, beispielsweise Oxidasen, Oxygenasen, Katalasen (die bei niedrigen H₂O₂ Konzentrationen als Peroxidase reagieren), Peroxidasen, wie Halo-, Chiaro-, Bromo-, Lignin-, Glucose- oder Manganperoxidasen, Dioxygenasen oder Laccasen (Phenoloxidasen, Polyphenoloxidasen) enthalten. Als geeignete Handelsprodukte sind Denilite® 1 und 2 der Firma Novozymes zu nennen. Als vorteilhaft einsetzbare Beispielsysteme für eine enzymatische Perhydrolyse wird auf die Anmeldungen WO 98/45398 A1, WO 2005/056782 A2 sowie WO 2004/058961 A1 verwiesen. Ein kombiniertes enzymatisches Bleichsystem, umfassend eine Oxidase und eine Perhydrolase beschreibt die Anmeldung WO 2005/124012. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluß zu gewährleisten (Mediatoren).

Die erfindungsgemäß eingesetzten Enzyme stammen entweder ursprünglich aus Mikroorganismen, etwa der Gattungen Bacillus, Streptomyces, Humicola, oder Pseudomonas, und/oder werden nach an sich bekannten biotechnologischen Verfahren durch geeignete Mikroorganismen produziert, etwa durch transgene Expressionswirte der Gattungen Bacillus oder durch filamentöse Pilze. Die Aufreinigung der betreffenden Enzyme erfolgt günstigerweise über an sich etablierte Verfahren, beispielsweise über Ausfällung, Sedimentation, Konzentrierung, Filtration der flüssigen Phasen, Mikrofiltration, Ultrafiltration, Einwirken von Chemikalien, Desodorierung oder geeignete Kombinationen dieser Schritte. Ferner können die Enzyme zusammen mit Begleitstoffen, etwa aus der Fermentation, oder mit Stabilisatoren konfektioniert werden.

Einen eigenen Erfindungsgegenstand stellt die Verwendung eines erfindungsgemäßen Wasch- oder Reinigungsmittels zur Entfernung von Anschmutzungen, insbesondere von protease-sensitiven Anschmutzungen, auf Textilien oder harten Oberflächen, d.h. zur Reinigung von Textilien oder von harten Oberflächen, dar. Denn Mittel können, insbesondere auf Grund der enthaltenen Kombination von Protease und Phosphonat, vorteilhaft dazu verwendet werden, um von Textilien oder von harten Oberflächen entsprechende Verunreinigungen zu beseitigen. Ausführungsformen dieses Erfindungsgegenstandes stellen beispielsweise die Handwäsche, die manuelle Entfernung von Flecken von Textilien oder von harten Oberflächen oder die Verwendung im Zusammenhang mit einem maschinellen Verfahren dar.

Alle Sachverhalte, Gegenstände und Ausführungsformen, die für Wasch- oder Reinigungsmittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

Einen weiteren Erfindungsgegenstand stellt ein Verfahren zur Reinigung von Textilien oder von harten Oberflächen dar, bei denen wenigstens in einem der Verfahrensschritte ein Wasch- oder Reinigungsmittel verwendet wird. Das Verfahren zur Reinigung von Textilien oder harten Oberflächen ist demnach dadurch gekennzeichnet, dass in mindestens einem Verfahrensschritt ein Wasch- oder Reinigungsmittel angewendet ist.

Hierunter fallen sowohl manuelle als auch maschinelle Verfahren, wobei maschinelle Verfahren aufgrund ihrer präziseren Steuerbarkeit, was beispielsweise die eingesetzten Mengen und Einwirkzeiten angeht, bevorzugt sind.

Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung bzw. Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines Wasch- oder Reinigungsmittels bereichert werden und stellen dann Ausführungsformen der vorliegenden Erfindung dar.

Alle Sachverhalte, Gegenstände und Ausführungsformen, die für Wasch- oder Reinigungsmittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch fürdie vorstehenden erfindungsgemäßen Verfahren gilt.

In einer bevorzugten Ausführungsform ist das Verfahren dadurch gekennzeichnet, dass das Phosphonat in der Waschflotte in einer Konzentration von 0,00075 bis 0,05 Gew.-% vorliegt und/oder dass die Protease in der Waschflotte in einer Konzentration von 0,0005 bis 0,03 Gew.-% vorliegt. Weitere bevorzugte Konzentrationen des in der Waschflotte vorliegenden Phosphonats sind von 0,00075 bis 0,01125 Gew.-%, von 0,001 bis 0,035 Gew.-%, von 0,002 bis 0,01125 Gew.-% oder von 0,00375 bis 0,0075 Gew.-%. Weitere bevorzugte Konzentrationen der in der Waschflotte vorliegenden Protease sind von 0,00075 bis 0,03 Gew.-% oder von 0,00077 bis 0,028 Gew.-%.

In einer weiteren bevorzugten Ausführungsform ist das Verfahren dadurch gekennzeichnet, dass es bei einer Temperatur zwischen 10°C und 60°C, zwischen 10°C und 55°C, zwischen 10°C und 50°C, zwischen 10°C und 40°C oder zwischen 20°C und 40°C durchgeführt wird.

In Mitteln eingesetzte Proteasen sind entsprechend der vorstehenden Ausführungen vorteilhaft in Wasch- und Reinigungsmitteln im Sinne der Erfindung sowie erfindungsgemäßen Verfahren, insbesondere Wasch- und Reinigungsverfahren, einsetzbar. Sie können also vorteilhaft dazu verwendet werden, um in entsprechenden Mitteln eine proteolytische Aktivität bereitzustellen.

Einen weiteren Gegenstand der Erfindung bildet daher die Verwendung einer Protease, (a1) die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Glutaminsäure (E) oder Asparaginsäure (D) aufweist, zur Bereitstellung einer proteolytischen Aktivität in einem flüssigen Wasch- oder Reinigungsmittel, welches ferner ein Phosphonat in einer Menge von 0,01 bis 4 Gew.-% umfasst.

Die Offenbarung umfasst auch die Verwendung einer Protease (a2) die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Asparagin (N) oder Glutamin (Q) aufweist, oder (a3) die eine Aminosäuresequenz umfasst, die zu der in SEQ ID NO. 1 angegebenen Aminosäuresequenz zu mindestens 80% identisch ist und die an Position 99 in der Zählung gemäß SEQ ID NO. 1 die Aminosäure Alanin (A) oder Glycin (G) oder Serin (S) aufweist, zur Bereitstellung einer proteolytischen Aktivität in einem flüssigen Wasch- oder Reinigungsmittel, welches ferner ein Phosphonat umfasst.

In einer weiteren Ausführungsform ist diese Verwendung dadurch gekennzeichnet, dass die Protease ferner in der Zählung gemäß SEQ ID NO. 1 mindestens eine der folgenden Aminosäuren aufweist:
(a) Threonin an Position 3 (3T),
(b) Isoleucin an Position 4 (41),
(c) Alanin, Threonin oder Arginin an Position 61 (61A, 61T oder 61R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (1991),
(h) Asparaginsäure, Glutaminsäure oder Glycin an Position 211 (211D, 211E oder 211G),
(i) Kombinationen der Aminosäuren (a) bis (h).

Einen weiteren Gegenstand der Erfindung bildet die Verwendung einer Protease, die ausgewählt ist aus der Gruppe bestehend aus
a. Protease umfassend eine Aminosäuresequenz gemäß SEQ ID NO. 2 oder SEQ ID NO. 3;
b. Protease, die eine gegenüber SEQ ID NO. 2 oder SEQ ID NO. 3 in mindestens einer Position veränderte Aminosäuresequenz umfasst, wobei die Veränderung in der Zählung gemäß SEQ ID NO. 1 ausgewählt ist aus der Gruppe bestehend aus:
   i. Threonin an Position 3 (3T),
   ii. Isoleucin an Position 4 (41),
   iii. Alanin, Threonin oder Arginin an Position 61 (61A, 61T oder 61R),
   iv. Asparaginsäure oder Glutaminsäure an Position 154 (154D oder 154E),
   v. Prolin an Position 188 (188P),
   vi. Methionin an Position 193 (193M),
   vii. Isoleucin an Position 199 (199I),
   viii. Asparaginsäure, Glutaminsäure oder Glycin an Position 211 (211D, 211E oder 211G),
   ix. Kombinationen der Aminosäuren (i) bis (viii);
zur Bereitstellung einer proteolytischen Aktivität in einem flüssigen Wasch- oder Reinigungsmittel, welches ferner ein Phosphonat in einer Menge von 0,01 bis 4 Gew.-% umfasst.

Ein weiteren Gegenstand der Offenbarung bildet die Verwendung einer Protease, die ausgewählt ist aus der Gruppe bestehend aus
a. Protease umfassend eine Aminosäuresequenz gemäß SEQ ID NO. 4 oder SEQ ID NO. 5 oder SEQ ID NO. 6 oder SEQ ID NO. 7 oder SEQ ID NO. 8;
b. Protease, die eine gegenüber SEQ ID NO. 4 oder SEQ ID NO. 5 oder SEQ ID NO. 6 oder SEQ ID NO. 7 oder SEQ ID NO. 8 in mindestens einer Position veränderte Aminosäuresequenz umfasst, wobei die Veränderung in der Zählung gemäß SEQ ID NO. 1 ausgewählt ist aus der Gruppe bestehend aus:
   i. Threonin an Position 3 (3T),
   ii. Isoleucin an Position 4 (41),
   iii. Alanin, Threonin oder Arginin an Position 61 (61A, 61T oder 61R),
   iv. Asparaginsäure oder Glutaminsäure an Position 154 (154D oder 154E),
   v. Prolin an Position 188 (188P),
   vi. Methionin an Position 193 (193M),
   vii. Isoleucin an Position 199 (1991),
   viii. Asparaginsäure, Glutaminsäure oder Glycin an Position 211 (211D, 211E oder 211G),
   ix. Kombinationen der Aminosäuren (i) bis (viii);
zur Bereitstellung einer proteolytischen Aktivität in einem flüssigen Wasch- oder Reinigungsmittel, welches ferner ein Phosphonat umfasst.

Alle Sachverhalte, Gegenstände und Ausführungsformen, die für Wasch- oder Reinigungsmittel beschrieben sind, sind auch auf die genannten Verwendungen anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verwendungen gilt.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme und Baukasten (Kits) wurden nach den Angaben der jeweiligen Hersteller eingesetzt.

### Beispiel 1

Ermittlung der Reinigungsleistung eines flüssigen Waschmittels

Für dieses Beispiel wurden standardisiert verschmutze Textilien eingesetzt, die von der EMPA Testmaterialien AG (St. Gallen, Schweiz), der wfk Testgewebe GmbH (Bruggen-Bracht, Deutschland) oder dem Center For Testmaterials (CFT, Vlaardingen, Niederlande) bezogen worden waren. Dabei wurden folgende Anschmutzungen und Textilien verwendet:
A: Gras auf Baumwolle: Produkt Nr. 164 von der Firma Eidgenössische Material- und Prüfanstalt (EMPA) Testmaterialien AG, St. Gallen, Schweiz;
B: Erdnuss Öl-Pigment/Tusche auf Polyester/Baumwolle: Produkt Nr. PC-10 von CFT (Center For Testmaterials) B.V. Vlaardingen, Niederlande;
C: Voll-Ei/Pigment (Ganzei/Ruß) auf Baumwolle: Produkt Nr. 1ON von der Firma wfk Testgewebe GmbH; Bruggen-Bracht, Deutschland;
D: Blut-Milch/Tusche auf Baumwolle: Produkt Nr. C-05 erhältlich von CFT (Center For Testmaterials) B.V. Vlaardingen, Niederlande

Mit diesem Testmaterial wurden verschiedene Waschmittel auf ihre Reinigungsleistung hin untersucht. Dafür wurden die Ansätze für 60 Minuten bei Temperaturen von 40°C bzw. 20°C gewaschen. Die Dosierung lag bei 7,4 Gramm des Waschmittels pro Liter Waschflotte. Es wurde mit Stadtwasser mit einer Wasserhärte von etwa 16° deutscher Härte gewaschen. Als Waschmittel diente eine Waschmittel-Basis-Rezeptur wie vorstehend in Tabelle 1 angegeben.

Diese Waschmittel-Basis-Rezeptur wurde für die verschiedenen Versuchsreihen Aktivitätsgleich (5 PE/ml Endkonzentration) mit folgenden Proteasen versetzt: Protease umfassend eine Aminosäuresequenz gemäß SEQ ID NO. 2 (Ansatz 1), leistungsverbesserte Variante F49 der Protease aus Bacillus lentus gemäß WO 95/23221 (Ansatz 2) und die in Figur 2 bzw. SEQ ID NO. 3 der internationalen Offenlegungsschrift WO 03/057713 offenbarte Protease (Ansatz 3).

Nach dem Waschen wurde der Weißheitsgrad der gewaschenen Textilien gemessen. Die Messung erfolgte an einem Spektrometer Minolta CM508d (Lichtart D65, 10°). Das Gerät wurde zuvor mit einem mitgelieferten Weißstandard kalibriert. Die erhaltenen Ergebnisse sind die Differenzremissionen zwischen einem Waschvorgang mit einem Waschmittel enthaltend eine Protease und einem parallel durchgeführten Kontrollwaschgang mit einem Waschmittel ohne Protease. Die Ergebnisse sind in der nachfolgenden Tabelle 2 zusammengestellt und erlauben einen unmittelbaren Rückschluss auf den Beitrag des jeweils enthaltenen Enzyms zur Reinigungsleistung des verwendeten Mittels.

**Tabelle 2: Waschergebnisse mit einem flüssigen Waschmittel bei 40°C bzw. 20°C**

| Anschmutzung | Ansatz 1 | | Ansatz 2 | | Ansatz 3 | |
|---|---|---|---|---|---|---|
| | 40°C | 20°C | 40°C | 20°C | 40°C | 20°C |
| A | 1,4 | 1,3 | 1,4 | 0,4 | 1,0 | 0,7 |
| B | 5,2 | 4,9 | 4,0 | 2,9 | 4,9 | 4,2 |
| C | 3,0 | 3,5 | 4,9 | 2,3 | 2,1 | 2,4 |
| D | 16,7 | 12,6 | 13,6 | 11,4 | 15,4 | 11,1 |

Es wird deutlich, dass ein Waschmittel im Sinne der Erfindung eine sehr gute und an den meisten Anschmutzungen sogar eine bessere Reinigungsleistung zeigt im Vergleich zu den Waschmitteln der Ansätze 2 und 3.

### Beispiel 2

Ermittlung der Reinigungsleistung eines flüssigen maschinellen Geschirrspülmittels

Gefäße mit harten, glatten Oberflächen wurden standardisiert mit den jeweiligen Anschmutzungen versehen und mit einer handelsüblichen Haushaltsgeschirrspülmaschine bei 40°C sowie 50°C gespült. Pro Spülgang wurden jeweils 30 ml eines flüssigen bzw. gelförmigen handelsüblichen maschinellen Geschirrspülmittels verwendet, das 2,4 Gew.-% Phosphonat (HEDP) enthielt. Es wurde mit Stadtwasser mit einer Wasserhärte von etwa 21° deutscher Härte gewaschen. Als Protease wurde Savinase ultra 16L (Novozymes, Ansatz 1) und eine erfindungsgemäße Protease umfassend eine Aminosäuresequenz gemäß SEQ ID NO. 2 (Ansatz 2) verwendet. Die Proteasen wurden gewichtsgleich eingesetzt bezogen auf Enzymprotein (0,68g Savinase ultra 16L bzw. die gewichtsgleiche Menge Enzymprotein der erfindungsgemal3.en Protease pro 30ml Reinigungsmittel).

Nach dem Spülen wurde der Abtrag der Anschmutzungen entweder gravimetrisch (Eigelb) oder visuell (weitere Anschmutzungen gemäß Tabelle 3) bestimmt. Fürdie gravimetrische Bestimmung wurde die Differenz aus dem Gewicht des verschmutzten und des gespülten Gefäßes bestimmt (freigesetztes Eigelb) und diese bezogen auf die Menge des ursprünglich aufgebrachten Eigelbs gemäß folgender Formel: % Reinigungsleistung = (mg freigesetztes Eigelb / mg aufgebrachtes Eigelb) x 100. Bei der visuellen Bestimmung wurde der Schmutzabtrag von diesbezüglich erfahrenen Personen visuell einer Prozentskala zugeordnet. Die Ergebnisse sind in der nachfolgenden Tabelle 3 zusammengestellt und erlauben einen unmittelbaren Rückschluss auf den Beitrag des jeweils enthaltenen Enzyms zur Reinigungsleistung des verwendeten Mittels.

**Tabelle 3: Reinigungsleistung eines erfindungsgemäßen flüssigen maschinellen Geschirrspülmittels bei 40°C bzw. 50°C**

| Anschmutzung | 40°C | | 50°C | |
|---|---|---|---|---|
| | Ansatz 1 | Ansatz 2 | Ansatz 1 | Ansatz 2 |
| Milch | 74 | 76 | 74 | 74 |
| Hackfleisch | 99 | 100 | 94 | 98 |
| Eigelb | 37 | 47 | 47 | 65 |

Es wird deutlich, dass ein flüssiges maschinelles Geschirrspülmittel im Sinne der Erfindung eine sehr gute und insbesondere an der Eigelb-Anschmutzung sogar eine deutlich bessere Reinigungsleistung zeigt im Vergleich zu dem Geschirrspülmittel gemäß Ansatz 1.

### Beispiel 3

Ermittlung der Lagerstabilität eines flüssigen Waschmittels

Die Waschmittel gemäß Ansatz 1 und 2 aus Beispiel 1 wurden hinsichtlich ihrer Lagerstabilität überprüft. Hierfür wurden die Waschmittel bei einer Temperatur von 30°C für den jeweils angegebenen Zeitraum gelagert und die jeweilige proteolytische Restaktivität über die Freisetzung des Chromophors para-Nitroanilin (pNA) aus dem Substrat bestimmt. Bei dem Substrat handelt es sich um suc-L-Ala-L-Ala-L-Pro-L-Phe-p-Nitroanilid (suc-AAPF-pNA). Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgte bei einer Temperatur von 25°C, bei pH 8,6 und einer Wellenlänge von 410 nm. Die Messzeit betrug 5 min. bei einem Messintervall von 20s bis 60s. Die erhaltenen Restaktivitäten sind in nachfolgender Tabelle 4 angegeben.

**Tabelle 4: Ermittlung der proteolytischen Restaktivität nach Lagerung**

| Waschmittel gemäß | Anfang | Woche 1 | Woche 2 |
|---|---|---|---|
| Ansatz 1 | 100% | 71% | 77% |
| Ansatz 2 | 100% | 47% | 37% |

Es wird deutlich, dass ein Waschmittel im Sinne der Erfindung eine deutlich verbesserte Lagerstabilität aufweist im Vergleich zu einem Waschmittel gemäß Ansatz 2. Das Waschmittel im Sinne der Erfindung weist nach einer Woche eine um 151% bzw. nach zwei Wochen eine um 211% erhöhte proteolytische Aktivität auf.

## Patentansprüche

1. Verwendung einer Protease,
(a1) die eine Aminosäuresequenz umfasst, die zu der in SEQ ID NO. 1 angegebenen Aminosäuresequenz zu mindestens 80% identisch ist und die an Position 99 in der Zählung gemäss SEQ ID NO. 1 die Aminosäure Glutaminsäure (E) oder Asparaginsäure (D) aufweist, zur Bereitstellung einer proteolytischen Aktivität in einem flüssigen Wasch- oder Reinigungsmittel, welches ferner ein Phosphonat in einer Menge von 0,01 bis 4 Gew.-% umfasst.

2. Verwendung nach Anspruch 1, **dadurch gekennzeichnet, dass** die Protease ferner in der Zählung gemäss SEQ ID NO. 1 mindestens eine der folgenden Aminosäuren aufweist:
(a) Threonin an Position 3 (3T),
(b) Isoleucin an Position 4 (41),
(c) Alanin, Threonin oder Arginin an Position 61 (61A, 61T oder 61R),
(d) Asparaginsäure oder Glutaminsäure an Position 154 (154D oder 154E),
(e) Prolin an Position 188 (188P),
(f) Methionin an Position 193 (193M),
(g) Isoleucin an Position 199 (1991),
(h) Asparaginsäure, Glutaminsäure oder Glycin an Position 211 (211D, 211E oder 211G),
(i) Kombinationen aus (a) bis (h).

3. Verwendung einer Protease, die ausgewählt ist aus der Gruppe bestehend aus
a. Protease umfassend eine Aminosäuresequenz gemäss SEQ ID NO. 2 oder SEQ ID NO. 3;
b. Protease, die eine gegenüber SEQ ID NO. 2 oder SEQ ID NO. 3 in mindestens einer Position veränderte Aminosäuresequenz umfasst, wobei die Veränderung in der Zählung gemäss SEQ ID NO. 1 ausgewählt ist aus der Gruppe bestehend aus:
i. Threonin an Position 3 (3T),
ii. Isoleucin an Position 4 (41),
iii. Alanin, Threonin oder Arginin an Position 61 (61A, 61T oder 61R),
iv. Asparaginsäure oder Glutaminsäure an Position 154 (154D oder 154E),
v. Prolin an Position 188 (188P),
vi. Methionin an Position 193 (193M),
vii. Isoleucin an Position 199 (1991),
viii. Asparaginsäure, Glutaminsäure oder Glycin an Position 211 (211D, 211E oder 211G),
ix. Kombinationen aus (i) bis (viii);
zur Bereitstellung einer proteolytischen Aktivität in einem flüssigen Wasch- oder Reinigungsmittel, welches ferner ein Phosphonat in einer Menge von 0,01 bis 4 Gew.% umfasst.

4. Verwendung eines Wasch- oder Reinigungsmittels umfassend eine Protease und ein Phosphonat, wie in einem der Ansprüche 1 bis 3 beschrieben, **dadurch gekennzeichnet, dass** das Phosphonat ausgewählt ist aus der Gruppe bestehend aus 1-Hydroxyethan-1,1-diphosphonsäure (HEDP), Aminotri(methylenphosphonsäure) (ATMP), Diethylentriaminpenta(methylenphosphonsäure) (DTPMP oder DETPMP oder DTPNT), Ethylendiamintetra(methylenphosphonsäure) (EDTMP), 2-Phosphonobutan-1,2,4-tricarbonsäure (PBS-AM) sowie Kombinationen hiervon.

5. Verwendung nach einem der Ansprüche 1 bis 4, **dadurch gekennzeichnet, dass** die Protease in einer Menge von 1x10⁻⁸ bis 5 Gewichts-Prozent, bezogen auf aktives Protein, enthalten ist.

6. Verwendung nach einem der Ansprüche 1 bis 5, **dadurch gekennzeichnet, dass** es ferner eine Komponente umfasst, die ausgewählt ist aus
i. anionische und/oder polyanionische Substanz, und/oder
ii. kationische und/oder polykationische Substanz, und/oder
iii. Hydroxyl- und/oder Polyhydroxyl-Gruppe(n) aufweisende Substanz.

7. Verwendung nach einem der Ansprüche 1 bis 6, **dadurch gekennzeichnet, dass** es mindestens einen weiteren Inhaltsstoff umfasst, insbesondere einen, der ausgewählt ist aus der Gruppe bestehend aus Tensid, Gerüststoff (Builder), Persauerstoffverbindung, Bleichaktivator, Alkohol, Säure, Vergrauungsinhibitor, optischer Aufheller, Schauminhibitor, wasserlösliches Salz, Verdickungsmittel, flüchtiges Alkali und/oder Base, hydrophilierendes Agens sowie Kombinationen hiervon.

8. Verwendung nach einem der Ansprüche 1 bis 7, **dadurch gekennzeichnet, dass** es mindestens ein weiteres Enzym umfasst, insbesondere eine Protease, Amylase, Cellulase, Hemicellulase, Mannanase, Tannase, Xylanase, Xanthanase, β-Glucosidase, Carrageenase, Perhydrolase, Oxidase, Oxidoreduktase oder eine Lipase, sowie vorzugsweise deren Gemische.

9. Verwendung nach einem der Ansprüche 1 bis 8 zur Entfernung von protease-sensitiven Anschmutzungen auf Textilien oder harten Oberflächen.

10. Verfahren zur Reinigung von Textilien oder harten Oberflächen, **dadurch gekennzeichnet, dass** in mindestens einem Verfahrensschritt ein Wasch- oder Reinigungsmittel wie in einem der Ansprüche 1 bis 8 definiert angewendet ist.

11. Verfahren nach Anspruch 10, **dadurch gekennzeichnet, dass** das Phosphonat in der Waschflotte in einer Konzentration von 0,00075 bis 0,05 Gew.-%, und/oder dass die Protease in der Waschflotte in einer Konzentration von 0,0005 bis 0,03 Gew.-% vorliegt.

12. Verfahren nach Anspruch 10 oder 11, **dadurch gekennzeichnet, dass** es bei einer Temperatur zwischen 10°C und 60°C, durchgeführt wird.

## Claims

1. The use of a protease
(a1) which comprises an amino acid sequence which is at least 80% identical to the amino acid sequence given in SEQ ID NO. 1 and which has at position 99 in the count according to SEQ ID NO. 1 the amino acid glutamic acid (E) or aspartic acid (D), for providing a proteolytic activity in a liquid washing or cleaning composition which also comprises a phosphonate in an amount of from 0.01 to 4% by weight.

2. The use according to claim 1, wherein the protease also has at least one of the following amino acids in the count according to SEQ ID NO. 1:
(a) threonine at position 3 (3T),
(b) isoleucine at position 4 (41),
(c) alanine, threonine or arginine at position 61 (61A, 61T or 61R),
(d) aspartic acid or glutamic acid at position 154 (154D or 154E),
(e) proline at position 188 (188P),
(f) methionine at position 193 (193M),
(g) isoleucine at position 199 (1991),
(h) aspartic acid, glutamic acid or glycine at position 211 (211D, 211E or 211G),
(i) combinations of (a) to (h).

3. The use of a protease which is selected from the group consisting of
a. protease comprising an amino acid sequence according to SEQ ID NO. 2 or SEQ ID NO. 3;
b. protease which comprises an amino acid sequence that is modified in at least one position compared to SEQ ID NO. 2 or SEQ ID NO. 3, where the modification is selected in the count according to SEQ ID NO. 1 from the group consisting of:
i. threonine at position 3 (3T),
ii. isoleucine at position 4 (41),
iii. alanine, threonine or arginine at position 61 (61A, 61T or 61R),
iv. aspartic acid or glutamic acid at position 154 (154D or 154E),
v. proline at position 188 (188P),
vi. methionine at position 193 (193M),
vii. isoleucine at position 199 (1991),
viii. aspartic acid, glutamic acid or glycine at position 211 (211D, 211E or 211G),
ix. combinations of (i) to (viii);
for providing a proteolytic activity in a liquid washing or cleaning composition which also comprises a phosphonate in an amount of from 0.01 to 4% by weight.

4. The use of a washing or cleaning composition comprising a protease and a phosphonate as described in any one of claims 1 to 3, wherein the phosphonate is selected from the group consisting of 1-hydroxyethane-1, 1-diphosphonic acid (HEDP), aminotri(methylenephosphonic acid) (ATMP), diethylenetriaminepenta(methylenephosphonic acid) (DTPMP or DETPMP or DTPNT) ethylenediaminetetra(methylenephosphonic acid) (EDTMP), 2-phosphonobutane-1,2,4-tricarboxylic acid (PBS-AM), and combinations thereof.

5. The use according to any one of claims 1 to 4, wherein the protease is present in an amount of from 1 x 10⁻⁸ to 5 percent by weight, based on active protein.

6. The use according to any one of claims 1 to 5, wherein it further comprises a component which is selected from
i. anionic and/or polyanionic substance, and/or
ii. cationic and/or polycationic substance, and/or
iii. substance having hydroxyl and/or polyhydroxyl group(s).

7. The use according to any one of claims 1 to 6, wherein it comprises at least one further ingredient, in particular one selected from the group consisting of surfactant, builder, peroxygen compound, bleach activator, alcohol, acid, graying inhibitor, optical brightener, foam inhibitor, water-soluble salt, thickener, volatile alkali and/or base, hydrophilizing agent, and combinations thereof.

8. The use according to any one of claims 1 to 7, wherein it comprises at least one further enzyme, in particular a protease, amylase, cellulase, hemicellulase, mannanase, tannase, xylanase, xanathanase, β-glucosidase, carrageenase, perhydrolase, oxidase, oxidoreductase or a lipase, and preferably mixtures thereof.

9. The use according to any one of claims 1 to 8 for removing protease-sensitive soilings on textiles or hard surfaces.

10. A process for cleaning textiles or hard surfaces, wherein a washing or cleaning composition as defined in any one of claims 1 to 8 is used in at least one process step.

11. The process according to claim 10, wherein the phosphonate is present in the wash liquor in a concentration of from 0.00075 to 0.05% by weight, and/or the protease is present in the wash liquor in a concentration of from 0.0005 to 0.03% by weight.

12. The process according to claim 10 or 11, wherein it is carried out at a temperature between 10°C and 60°C.

## Revendications

1. Utilisation d'une protéase
(a1) qui présente une séquence d'acides aminés qui a une identité d'au moins 80 % avec la séquence d'acides aminés indiquée dans SEQ ID N° 1 et qui en position 99 dans la numérotation selon SEQ ID N° 1 présente l'acide aminé acide glutamique (E) ou acide asparagique (D),
pour la mise à disposition d'une activité protéolytique dans une lessive ou un détergent liquide qui comprend en outre un phosphonate en une quantité de 0,01 à 4 % en poids.

2. Utilisation selon la revendication 1, **caractérisée en ce que** la protéase présente en outre, dans la numérotation selon SEQ ID N° 1, au moins l'un des acides aminés suivantes :
(a) une thréonine en une position 3 (3T),
(b) une isoleucine en position 4 (4I),
(c) une alanine, une thréonine ou une arginine en position 61 (61A, 61T ou 61R),
(d) un acide asparagique ou un acide glutamique en position 154 (154D ou 154E),
(e) une proline en position 188 (188P),
(f) une méthionine en position 193 (193M),
(g) une isoleucine en position 199 (199I),
(h) un acide asparagique, un acide glutamique ou une glycine en position 211 (211D, 211E ou 211G),
(i) des combinaisons de (a) à (h).

3. Utilisation d'une protéase qui est choisie dans le groupe consistant en
a. une protéase comprenant une séquence d'acides aminés selon SEQ ID N° 2 ou SEQ ID N° 3,
b. une protéase qui comprend une séquence d'acides aminés modifiée au niveau d'au moins une position par rapport à SEQ ID N° 2 ou SEQ ID N° 3, la modification dans la numérotation selon SEQ ID N° 1 étant choisie dans le groupe consistant en :
i. une thréonine en position 3 (3T),
ii. une isoleucine en position 4 (4I),
iii. une alanine, une thréonine ou une arginine en position 61 (61A, 61T ou 61R),
iv. un acide asparagique ou un acide glutamique en position 154 (154D ou 154E),
v. une proline en position 188 (188P),
vi. une méthionine en position 193 (193M),
vii. une isoleucine en position 199 (199I),
viii. un acide asparagique, un acide glutamique ou une glycine en position 211 (211D, 211E ou 211G),
ix. les combinaisons de (i) à (viii) ;
pour la mise à disposition d'une activité protéolytique dans une lessive ou un détergent liquide, qui comprend en outre un phosphonate en une quantité de 0,01 à 4 % en poids.

4. Utilisation d'une lessive ou d'un détergent comprenant une protéase et un phosphonate tel que décrit dans l'une des revendications 1 à 3, **caractérisée en ce que** le phosphonate est choisi dans le groupe consistant en l'acide 1-hydroxyéthane-1,1-diphosphonique (HEDP), l'acide aminotri(méthylènephosphonique) (ATMP), l'acide diéthylènetriaminepenta(méthylènephosphonique) (DTPMP ou DETPMP ou DTPNT), l'acide éthylènédiaminetétra(méthylènephosphonique) (EDTMP), l'acide 2-phosphonobutane-1,2,4-tricarboxylique (PBS-AM), ainsi que les combinaisons de ceux-ci.

5. Utilisation selon l'une des revendications 1 à 4, **caractérisée en ce que** la protéase est présente en une quantité de 1 x 10⁻⁸ à 5 % en poids, par rapport à la protéine active.

6. Utilisation selon l'une des revendications 1 à 5, **caractérisée en ce qu'**il comprend en outre un composant qui est choisi parmi
i. une substance anionique et/ou polyanionique, et/ou
ii. une substance cationique et/ou polycationique, et/ou
iii. une substance comprenant un ou plusieurs groupes hydroxyle et/ou polyhydroxyle.

7. Utilisation selon l'une des revendications 1 à 6, **caractérisée en ce qu'**il comprend au moins un constituant supplémentaire, qui est choisi dans le groupe consistant en un tensioactif, un adjuvant (builder), un composé peroxygéné, un activateur de blanchiment, un alcool, un acide, un inhibiteur de grisaillement, un azurant optique, un inhibiteur de mousse, un sel soluble dans l'eau, un épaississant, un alcali et/ou une base volatils, un agent d'hydrophilisation, ainsi que les combinaisons de ceux-ci

8. Utilisation selon l'une des revendications 1 à 7, **caractérisée en ce qu'**il comprend au moins une enzyme supplémentaire, en particulier une protéase, une amylase, une cellulase, une hémicellulase, une mannanase, une tannase, une xylanase, une xanthanase, une β-glucosidase, une carragénase, une perhydrolase, une oxydase, une oxydoréductase ou une lipase, ainsi que de préférence des mélanges de celles-ci.

9. Utilisation selon l'une des revendications 1 à 8 pour éliminer les salissures sensibles aux protéases sur des textiles ou des surfaces dures.

10. Procédé pour le nettoyage de textiles ou de surfaces dures, **caractérisé en ce que**, dans au moins une étape du procédé, on utilise une lessive ou un détergent tel que défini dans l'une des revendications 1 à 8.

11. Procédé selon la revendication 10, **caractérisé en ce que** le phosphonate est présent dans le bain de lavage en une concentration de 0,00075 à 0,05 % en poids, et/ou que la protéase est présente dans le bain de lavage en une concentration de 0,0005 à 0,03 % en poids.

12. Procédé selon la revendication 10 ou 11, **caractérisé en ce qu'**il est mis en oeuvre à une température comprise entre 10 et 60°C.
